# EUROPEAN PATENT SPECIFICATION

(11) **EP 3 519 058 B1**
(45) Date of publication and mention of the grant of the patent: **07.06.2023**
(21) Application number: 17781354.0
(22) Date of filing: 29.09.2017
(51) Int. Cl.: A61Q 5/06, A61K 8/34, A61K 8/37, A61K 8/60, A61K 8/81, A61K 8/86, A61K 8/87, A61K 8/898, A61Q 1/10

(54) **COMPOSITIONS AND METHODS FOR TREATING HAIR**
ZUSAMMENSETZUNGEN UND VERFAHREN ZUR BEHANDLUNG VON HAAR
COMPOSITIONS ET MÉTHODES DE TRAITEMENT DES CHEVEUX

(30) Priority: 30.09.2016 US 201615282502
(43) Date of publication of application: 07.08.2019
(73) Proprietor: L'OREAL, 75008 Paris (FR); Comeron, Vanessa, Clark, NJ 07066 (US)
(72) Inventor: COMERON, Vanessa, Clark NJ 07066 (US); SULEIMAN, Aziza, Clark New Jersey 07066 (US); MAHADESHWAR, Anand, Clark New Jersey 07066 (US)
(74) Representative: Casalonga
(86) International application number: PCT/US2017/054364
(87) International publication number: WO 2018/064511

(56) References cited:
- WO-A1-2013/064596
- WO-A1-2014/210480
- US-A1- 2015 004 114
- US-A1- 2015 004 116
- US-A1- 2015 004 119
- US-A1- 2015 004 121
- US-A1- 2016 175 237
- US-A1- 2016 184 195

## Description

### FIELD OF THE INVENTION

The disclosure relates to compositions for use on keratinous substances. In particular, it relates to a composition and methods for styling the hair.

### BACKGROUND

Compositions for styling the hair are known, such as, for example, hair spray compositions, hair gels and mousses, hair volumizing compositions, hair smoothing creams, lotions, serums, oils, clays. The goals of many hair styling compositions include holding or fixing the hair in a particular shape, imparting or increasing volume of the hair, smoothing the hair, and/or decreasing or eliminating the appearance of frizz.

Drawbacks associated with current products for styling the hair include that the product can be sticky or tacky and/or can produce a film that imparts a sticky or tacky feel. Moreover, hair styled with current products can be stiff and/or "crunchy" in that the film is hard and brittle resulting in a crunching feel or sound when the hair is touched, which is undesirable for most consumers.

Current products for styling the hair typically include water-soluble film-forming polymers. Depending on the chemical make-up of these polymers, they may be either soluble in water, or they may be water insoluble polymers which are made water soluble via various chemical modifications, such as neutralization. Solutions comprising these polymers tend to be viscous, and often as the concentration of the polymer increases, its viscosity rapidly increases. In styling applications, as the solvent evaporates, the polymer solution becomes thicker on the hair surface, resulting in a sticky or tacky film. These products also tend to exhibit problems with product spreadability, hair manageability, and humidity resistance, which is especially a problem in hot and humid environments.

Particularly, while previous compositions comprising latex polymers may provide clean properties to the hair given its anionic nature, the clean properties can translate into difficult application and/or distribution of the product, quick absorption, dryness, and/or possibly static in the hair. Often, other ingredients may be used in combination with latex polymers to overcome the brittleness and stiffness that may result from the use of latex polymers in hair compositions. However, it can still be challenging for manufacturers to incorporate new ingredients into the compositions because this may negatively impact performance, certain cosmetic attributes, texture, and formulation stability.

Compositions comprising latex polymers are described in particular in US 2015/004116, WO 2014/210480, US 2015/004114, US 2016/175237 and US 2016/184195.

It has now been surprisingly and unexpectedly discovered that combining at least two latex polymers, wherein at least one latex polymer is a film-forming polymer, with at least one specific aminofunctional silicone, at least one thickening agent chosen from optionally crosslinked and/or neutralized 2-acrylamido-2-methylpropanesulfonic acid polymers (AMPS) and copolymers, crosslinked anionic copolymers of acrylamide, crosslinked anionic copolymers of AMPS, emulsions of crosslinked anionic copolymers of acrylamide/nonionic surfactants, emulsions of AMPS/nonionic surfactants, or mixtures thereof, at least one nonionic surfactant chosen from glyceryl esters, fatty alcohols, alkoxylated alcohols and lanolin, alkylpolyglucosides, or mixtures thereof, produces a composition that exhibits excellent cosmetic properties in wet and dry stages of hair while maintaining desirable care benefits and styling and shaping properties. Such compositions can allow for a clean, natural, and/or "invisible" feel; a lack of stickiness; frizz control; high humidity resistance; curl and/or wave definition; and styling hold even when heat is not used for drying and/or shaping and styling the hair. These compositions may be useful in hair-styling applications wherein styling benefits such as natural look, curling or straightening, and/or various degrees of styling hold are imparted to hair.

### SUMMARY

The disclosure relates, in various embodiments, to a cosmetic composition comprising at least two latex polymers, wherein at least one latex polymer is a film-forming polymer; at least one aminofunctional silicone chosen from amodimethicone/morpholinomethyl silsesquioxane copolymer, PEG-40/PPG-8 methylaminopropyl/hydroxypropyl dimethicone copolymer, trideceth-9 PG-amodimethicone, or mixtures thereof, and present in an amount ranging from 0.25% to 3% by weight relative to the total weight of the composition; at least one thickening agent chosen from optionally crosslinked and/or neutralized 2-acrylamido-2-methylpropanesulfonic acid polymers (AMPS) and copolymers, crosslinked anionic copolymers of acrylamide, crosslinked anionic copolymers of AMPS, emulsions of crosslinked anionic copolymers of acrylamide/nonionic surfactants, emulsions of AMPS/nonionic surfactants, or mixtures thereof; at least one nonionic surfactant chosen from glyceryl esters, fatty alcohols, alkoxylated alcohols and lanolin, alkylpolyglucosides, or mixtures thereof; and water; wherein the at least two latex polymers are present in a combined amount ranging from 0.1% to 30% by weight, relative to the weight of the composition.

In various embodiments, the at least two latex polymers in the composition of the present invention are present in a combined amount ranging from 0.25% to 20% by weight, or from 0.3 % to 15% by weight, or from 0.4 % to 10% by weight, or from 0.5% to 5% by weight, relative to the total weight of the composition.

In an embodiment, the at least two latex polymers in the composition of the present invention comprise at least one acrylate latex polymer and at least one polyurethane latex polymer.

In an embodiment, the at least two latex polymers in the composition of the present invention comprise acrylates copolymer and polyurethane-34.

In an embodiment, the weight ratio of the at least one acrylate latex polymer to the at least one polyurethane latex polymer ranges from 10:1 to 1:10 or from 8:1 to 1:8 or from 6:1 to 1:6 or from 5:1 to 1:5 or from 2:1 to 1:2.

In an embodiment, the weight ratio of the at least one acrylate latex polymer to the at least one polyurethane latex polymer is equal to or less than 1.

In an embodiment, the weight ratio of the at least one acrylate latex polymer to the at least one polyurethane latex polymer is 1.

In an embodiment, the weight ratio of the at least one acrylate latex polymer to the at least one polyurethane latex polymer is equal to or more than 1.

In an embodiment, the weight ratio of the at least one acrylate latex polymer to the at least one polyurethane latex polymer is equal to or more than 2.

In an embodiment, the at least one aminofunctional silicone is present in an emulsion comprising nonionic surfactants.

In an embodiment, the at least one thickening agent is chosen from a water-in-oil emulsion of Polyacrylamide/C13-14 Isoparaffin/Laureth-7, a water-in-oil emulsion of Acrylamide/Sodium acryloyldimethyltaurate copolymer/lsohexadecane/Polysorbate 80, or mixtures thereof.

In an embodiment, the at least one nonionic surfactant includes glyceryl esters chosen from glyceryl oleate, glyceryl monostearate (or glyceryl stearate), glyceryl monoisostearate, glyceryl monopalmitate, glyceryl monobehenate, or mixtures thereof.

In an embodiment, the at least one nonionic surfactant is chosen from non-alkoxylated, saturated or unsaturated, linear or branched fatty alcohols having from 6 to 60 carbon atoms, or mixtures thereof.

In an embodiment, the at least one nonionic surfactant is chosen from cetyl alcohol, stearyl alcohol, cetearyl alcohol (mixture of cetyl alcohol and stearyl alcohol), PEG-40 hydrogenated castor oil, PEG-75 lanolin, laureth-7, laureth-12, trideceth-10, trideceth-12, cetearyl glucoside, decyl glucoside, lauryl glucoside, stearyl glucoside, coco-glucoside, or mixtures thereof.

In one embodiment, the cosmetic composition of the present invention comprises:
at least one acrylate latex polymer;
at least one polyurethane latex polymer;
at least one aminofunctional silicone chosen from amodimethicone/morpholinomethyl silsesquioxane copolymer, PEG-40/PPG-8 methylaminopropyl/hydroxypropyl dimethicone copolymer, trideceth-9 PG-amodimethicone, or mixtures thereof and present in an amount ranging from 0.25% to 3% by weight;

at least one thickening agent chosen from agent chosen from optionally crosslinked and/or neutralized 2-acrylamido-2-methylpropanesulfonic acid polymers (AMPS) and copolymers, crosslinked anionic copolymers of acrylamide, crosslinked anionic copolymers of AMPS, emulsions of crosslinked anionic copolymers of acrylamide/nonionic surfactants, emulsions of AMPS/nonionic surfactants, or mixtures thereof, and present in an amount ranging from 0.25% to 6% by weight;
at least one nonionic surfactant chosen from glyceryl esters, fatty alcohols, alkoxylated alcohols and lanolin, alkylpolyglucosides, or mixtures thereof and present in an amount ranging from 0.2% to 5% by weight; and water;

all weights being relative to the total weight of the composition.
wherein the weight ratio of the at least one acrylate latex polymer to the at least one polyurethane latex polymer is equal to or less than 1.

In one embodiment, the at least one aminofunctional silicone comprises PEG-40/PPG-8 methylaminopropyl/hydroxypropyl dimethicone copolymer and trideceth-9 PG-amodimethicone.

In one embodiment, the at least one aminofunctional silicone is trideceth-9 PG-amodimethicone.

In an embodiment, the cosmetic composition of the present invention further comprises at least one non-latex nonionic film forming polymer chosen from:
- polyalkyloxazolines;
- vinyl acetate homopolymers;
- vinyl acetate copolymers;
- homopolymers and copolymers of acrylic esters;
- copolymers of acrylonitrile and a nonionic monomer;
- styrene homopolymers;
- styrene copolymers, for instance copolymers of styrene and of an alkyl (meth)acrylate; copolymers of styrene, of alkyl methacrylate and of alkyl acrylate; copolymers of styrene and of butadiene; or copolymers of styrene, of butadiene and of vinylpyridine;
- polyamides;
- vinylpyrrolidone homopolymers;
- copolymer of vinylpyrrolidone and vinyl acetate monomers;
- vinyllactam homopolymers including and polyvinylcaprolactam; and
- vinyllactam copolymers including poly(vinylpyrrolidone/vinyllactam) copolymer, poly(vinylpyrrolidone/vinyl acetate) copolymers; poly(vinylpyrrolidone/vinyl acetate/vinyl propionate) terpolymers, or mixtures thereof.

In one embodiment, the at least one non-latex nonionic film forming polymer is chosen from VPNA copolymer (or PVP/VA copolymer), PVP, or mixtures thereof.

In one embodiment, the at least one non-latex nonionic film forming polymer is present in an amount ranging from 0.05% to 15% by weight, or from 0.1% to 10% by weight, or from 0.5% to 5% by weight, relative to the total weight of the composition.

In an embodiment, the cosmetic composition of the present invention further comprises an additional thickening agent, present in a total amount ranging from 0.05% to 10% by weight, and chosen from polysaccharides, gums, guar gums, celluloses, glucans, silicas or hydrophobic silicas, nonionic homopolymers or copolymers containing ethylenically unsaturated monomers of the amide type, modified or unmodified carboxyvinyl polymers, sodium salts of polyhydroxycarboxylic acids, polymers comprising at least one hydrophilic unit and at least one fatty-chain allyl ether unit, a copolymer of ammonium acryloyldimethyltaurate and vinylpyrrolidone monomers;or mixtures thereof, and preferably, xanthan gum, guar gum, hydroxypropyl guar, guar hydroxypropyl trimonium chloride, hydroxyethyl cellulose, hydroxypropyl cellulose, cetyl hydroxyethyl cellulose, hydroxypropyl starch phosphate, ammonium acryloyldimethyltaurate/VP copolymer or mixtures thereof.

In one embodiment, the cosmetic composition according to the invention, further comprises at least one coalescing agent and/or plasticizer, present in a total amount ranging from 0.1% to 20% by weight, relative to the total weight of the composition.

In an embodiment, the cosmetic composition of the present invention, the at least one coalescing agent and/or plasticizer is chosen from glycol ethers, glycol esters, sucrose esters, propylene glycol ethers, propylene glycol esters, propylene glycol dibenzoate, dipropylene glycol dibenzoate, propylene glycol butyl ether, or mixtures thereof.

In an embodiment, the cosmetic composition of the present invention further comprises a fatty ester.

In one embodiment, the cosmetic composition according to the invention, further comprises at least one propellant.

In one embodiment, the cosmetic composition according to the invention, further comprises at least one cosmetically acceptable organic solvent chosen from volatile and non-volatile solvents.

In an embodiment, the cosmetic composition of the present invention further comprises at least one additional component chosen from anionic surfactants, cationic surfactants, cationic polymers, organic amines, carbonate compounds, emulsifying agents, fillers, pigments, conditioning agents, moisturizing agents, shine agents, sequestering agents, fragrances, preservatives, pH modifiers/neutralizing agents, stabilizers, salts, or mixtures thereof.

In various embodiments, any of the above-described cosmetic compositions of the present invention is in the form of a cream or a thick lotion or a gel or an oil-gel.

In one embodiment, the present invention is directed to a method of styling or shaping hair, the method comprising applying to the hair anyone of the compositions of the invention,

In one embodiment, the method of the present invention includes a step of air drying the hair after applying the composition to the hair.

In one embodiment, the method of the present invention does not include treating the hair with heat during or after applying the composition onto the hair.

In one embodiment, the method of the present invention further comprises a step of treating the hair with heat at a temperature ranging from 25°C to 250°C before, during, or after the application of said composition.

Additional features and advantages of the disclosed embodiments as claimed will be set forth in the detailed description which follows, and in part will be readily apparent to those skilled in the art from that description or recognized by practicing the invention as claimed herein, including the detailed description which follows, as well as the claims.

It is to be understood that both the foregoing general description and the following detailed description present various embodiments of the disclosure, and are intended to provide an overview or framework for understanding the nature and character of the claims.

### LATEX POLYMERS

The compositions according to the disclosed embodiments comprise at least two latex polymers, wherein at least one latex polymer is a film-forming polymer.

In various embodiments, the latex polymers may be identified as polymer A and polymer B. Compositions according to certain embodiments may comprise at least one polymer A and at least one polymer B, wherein at least one of polymer A and polymer B is a film-forming polymer.

According to some embodiments, polymer A may be chosen from latex polymers having a Young's modulus ranging from 0.1 MPa to 10 MPa and a strain, under stress at 0.5 MPa, of at least 1%; and polymer B may be chosen from latex polymers having a Young's modulus ranging from 10 MPa to 6 GPa and a strain, under stress at 0.5 MPa, of less than 5%.

In at least certain embodiments, polymer A may have a glass transition temperature (Tg) ranging from -90°C to 40°C, and polymer B may have a glass transition temperature (Tg) ranging from 40°C to 200°C.

According to some embodiments, polymers A and B may be chosen from acrylate and polyurethane polymers, with the proviso that when polymer A is chosen from an acrylate polymer, polymer B is chosen from a polyurethane polymer; and when polymer A is chosen from a polyurethane polymer, polymer B is chosen from an acrylate polymer.

In some embodiments, latex polymers A and B may be chosen such that polymer A comprises at least one latex polymer which is optionally a film-forming polymer that is a relatively soft, flexible latex polymer, and polymer B comprises at least one latex polymer which is optionally a film-forming polymer that is a relatively hard, brittle polymer

At least one of polymer A and polymer B is a film-forming polymer. In various embodiments, latex polymer A is a film-forming polymer and latex polymer B is a non-film-forming polymer. In other embodiments, latex polymer A is a non-film-forming polymer and latex polymer B is a film-forming polymer. In further embodiments, both latex polymer A and latex polymer B are film-forming polymers.

As used herein, a film-forming polymer is meant to include a polymer that is capable, by itself or in the presence of an auxiliary film-forming agent, of forming a macroscopically continuous film that adheres to keratin materials, and preferably a cohesive film, better still, a film whose cohesion and mechanical properties are such that said film can be isolated and manipulated individually, for example, when said film is prepared by pouring onto a non-stick surface such as Teflon-coated or silicone-coated surface. In addition, as used herein, a non-film-forming polymer is meant to include a polymer which will not form a film at or below ambient temperature, or in other words, will only form a film at temperatures above ambient. For purposes of this disclosure, ambient temperature is below 40°C, such as ranging from 15°C to 30°C.

In various embodiments, the latex polymers are provided in the form of aqueous dispersions prior to formulating the compositions of the disclosure. In other embodiments, the aqueous dispersions may be obtained through an emulsion polymerization of monomers wherein the resulting latex polymers have a particle size less than 1µm. In certain other embodiments, a dispersion prepared by the polymerization in water of one or more monomers having a polymerizable double bond may be chosen. In other embodiments, the latex polymers are produced from condensation reactions between monomers and subsequently dispersed in an aqueous medium.

In various embodiments, the latex polymers may exist as dispersed polymer particles in a dispersion medium, such as an aqueous dispersion medium. In other embodiments, the latex polymers may be dispersed in independent dispersion media. In further embodiments, the latex polymers may be dispersed together in the same dispersion medium.

The dispersion medium comprises at least one solvent chosen from water. The dispersion medium may further comprise at least one solvent chosen from cosmetically acceptable organic solvents. Cosmetically acceptable organic solvents may, in various embodiments, be water-miscible, e.g. capable of forming at 25°C a homogeneous mixture that is transparent, or substantially transparent, to the eye. For instance, cosmetically acceptable organic solvents may be chosen from lower monoalcohols, such as those containing from 1 to 5 carbon atoms, for example ethanol and isopropanol; polyols, including glycols, such as those containing from 2 to 8 carbon atoms, for example propylene glycol, ethylene glycol, 1,3-butylene glycol, dipropylene glycol, hexylene glycol, and glycerin; hydrocarbons, for example, isododecane and mineral oil; and silicones, for example dimethicones, cyclic dimethicones (INCI name: cyclomethicones), and cyclopentasiloxane; or mixtures thereof.

In other embodiments, the solvent of the dispersion medium comprises water. In other embodiments, the solvent of the dispersion medium comprises water and at least one cosmetically acceptable organic solvent. In further embodiments, the solvent comprises water. In further embodiments, the solvent of the dispersion medium primarily consists essentially of water. For example, the solvent of the dispersion medium may, in at least certain exemplary embodiments, comprise greater than 50% water, greater than 55% water, greater than 60% water, greater than 65% water, greater than 70% water, greater than 75% water, greater than 80% water, greater than 85% water, greater than 90% water, greater than 95% water, greater than 96% water, greater than 97% water, greater than 98% water, or greater than 99% water.

In various embodiments, the latex polymer particles are not soluble in the solvent of the dispersion medium, i.e. are not water soluble and/or are not soluble in the at least one cosmetically acceptable organic solvent. Accordingly, the latex polymers retain their particulate form in the solvent or solvents chosen.

In another embodiment, the aqueous dispersions obtained through an emulsion polymerization may be spray-dried.

In certain embodiments, latex particles according to the disclosure may have an average diameter ranging up to 1000 nm, from 50 nm to 800 nm, or from 100 nm to 500 nm. Such particle sizes may be measured with a laser granulometer (e.g. Brookhaven BI90).

In various embodiments, the latex polymers may, independently, be neutralized, partially neutralized, or unneutralized. In other embodiments where the latex polymers are neutralized or partially neutralized, the particle size may be, for example, greater than 800 nm. In certain embodiments, the particulate form of the latex polymers is retained in the dispersion medium.

In various embodiments, the latex polymers may be chosen from uncharged and charged latex polymers. In other embodiments, the latex polymers may be chosen from nonionic latex polymers, cationic latex polymers, and anionic latex polymers.

In some embodiments, the latex polymers may be chosen, independently, from acrylate latex polymers and polyurethane latex polymers. As described herein, it is to be understood that when latex polymer A is chosen from an acrylate polymer, latex polymer B is chosen from a polyurethane polymer; and when latex polymer A is chosen from a polyurethane polymer, latex polymer B is chosen from an acrylate polymer.

In certain embodiments, one of the at least two latex polymers may be chosen from acrylate latex polymers, such as those resulting from the homopolymerization or copolymerization of monomers chosen from (meth)acrylics, (meth)acrylates, (meth)acrylamides and/or vinyl homopolymers or copolymers. The term "(meth)acryl" and variations thereof, as used herein, means acryl or methacryl.

In certain embodiments, the (meth)acrylic monomers may be chosen from acrylic acid, methacrylic acid, citraconic acid, itaconic acid, maleic acid, fumaric acid, crotonic acid, maleic anhydride, or mixtures thereof.

In certain embodiments, the (meth)acrylic monomers may be chosen from C1-C8 alkyl (meth)acrylic, methyl (meth)acrylic, ethyl (meth)acrylic, propyl (meth)acrylic, isopropyl (meth)acrylic, butyl (meth)acrylic, tert-butyl (meth)acrylic, pentyl(meth) acrylic, isopentyl (meth)acrylic, neopentyl (meth)acrylic, hexyl (meth)acrylic, isohexyl (meth)acrylic, 2-ethylhexyl (meth)acrylic, cyclohexyl (meth)acrylic, isohexyl (meth)acrylic, heptyl (meth)acrylic, isoheptyl (meth)acrylic, octyl (meth)acrylic, isooctyl (meth)acrylic, or mixtures thereof.

In certain embodiments, the esters of (meth)acrylic monomers may be chosen from C1-C8 alkyl (meth)acrylate, methyl (meth)acrylate, ethyl (meth)acrylate, propyl (meth)acrylate, isopropyl (meth)acrylate, butyl (meth)acrylate, tert-butyl (meth)acrylate, pentyl(meth) acrylate, isopentyl (meth)acrylate, neopentyl (meth)acrylate, hexyl (meth)acrylate, isohexyl (meth)acrylate, 2-ethylhexyl (meth)acrylate, cyclohexyl (meth)acrylate, isohexyl (meth)acrylate, heptyl (meth)acrylate, isoheptyl (meth)acrylate, octyl (meth)acrylate, isooctyl (meth)acrylate, allyl (meth)acrylate, or combinations thereof.

In certain embodiments, the esters of (meth)acrylic monomers may be chosen from C1-C8 alkoxy (meth)acrylate, methoxy (meth)acrylate, ethoxy (meth)acrylate, propyl oxide (meth)acrylate, isopropyl oxide (meth)acrylate, butyl oxide (meth)acrylate, tert-butyl oxide (meth)acrylate, pentyl oxide (meth) acrylate, isopentyl oxide (meth)acrylate, neopentyl oxide (meth)acrylate, C2-C6 hydroxy alkyl (meth)acrylates, hydroxy ethyl (meth)acrylate, 2-hydroxypropyl (meth)acrylate, glycidyl (meth)acrylate, ethylene glycol di(meth)acrylate, polyethylene glycol mono(meth)acrylate, 1,4-butane diol di(meth)acrylate, 1,6,hexane diol di(meth)acrylate, aryl (meth)acrylates benzyl (meth)acrylate, phenyl (meth)acrylate, or mixtures thereof.

In certain embodiments, the esters can further contain amino groups such as aminoethyl (meth)acrylate, N,N-dimethylaminoethyl (meth)acrylate, N,N-dimethylaminopropyl (meth)acrylate, N,N-dimethylaminodimethylpropyl (meth)acrylate, N,N-diethyleaminoethyl (meth)acrylate, N,N,N-trimethylaminoethyl (meth)acrylate, salts of the ethylenic amines, or silicone macromonomers.

In various embodiments, the alkyl group of the esters may be either fluorinated or perfluorinated, for example one, some, or all of the hydrogen atoms of the alkyl group are substituted with fluorine atoms. In other embodiments, the monomers can also be fluorine-containing monomers, such as trifluoroethyl methacrylate, 2,2,3,3-tetrafluoropropyl methacrylate, 2,2,3,3,4,4-hexafluorobutyl methacrylate, perfluorooctyl methacrylate, or perfluorooctyl acrylate.

In certain embodiments, the amides of (meth)acrylic monomers can, for example, be made of (meth)acrylamide, N-alkyl (meth)acrylamides, N-(C1-C12) alkyl (meth)acrylates such as N-ethyl (meth)acrylamide, N-t-butyl (meth)acrylamide, N-t-octyl (meth)acrylamide, N-methylol (meth)acrylamide, N-diacetone (meth)acrylamide, or mixtures thereof.

In certain embodiments, the vinyl monomers can include, but are not limited to, vinyl cyanide compounds such as acrylonitrile and methacrylonitrile; vinyl esters such as vinyl formate, vinyl acetate, vinyl propionate, vinyl neodecanoate, vinyl pivalate, vinyl benzoate, vinyl t-butyl benzoate, and triallyl cyanurate; vinyl halides such as vinyl chloride and vinylidene chloride; aromatic mono- or divinyl compounds such as styrene, α-methylstyrene, chlorostyrene, alkylstyrene, divinylbenzene and diallyl phthalate; or mixtures thereof. In other embodiments, the vinyl mononers can include para-styrensulfonic, vinylsulfonic, 2-(meth)acryloyloxyethylsulfonic, or 2-(meth)acrylamido-2-methylpropylsulfonic acids.

The list of monomers given is not limiting, and it should be understood that it is possible to use any monomer known to those skilled in the art which includes acrylic and/or vinyl monomers (including monomers modified with a silicone chain).

In certain embodiments, silicone acrylic polymers may also optionally be used as vinyl polymer in at least one exemplary and non-limiting embodiment.

In certain embodiments, acrylic latex polymers may be chosen from aqueous dispersions of Methacrylic Acid/Ethyl Acrylate copolymer (INCI name: Acrylates Copolymer, such as Luviflex^{®} Soft sold by the company BASF), PEG/PPG-2316 Dimethicone Citraconate/C10-30 Alkyl PEG-25 Methacrylate/Acrylic Acid/Methacrylic Acid/Ethyl Acryiate/Trimethylolpropane PEG-15 Triacrylate copolymer (INCI name: Polyacrylate-2 Crosspolymer, such as Fixate^{™} Superhold sold by the company Lubrizol), Butyl acrylate, PEG-10 acrylate, PPG-6 acrylate and dimethylacrylamide copolymer (INCI name: Polyacrylate-3 crosspolymer), Styrene/Acrylic copolymer (such as Neocryl^{®} A-1120 sold by the company DSM), Ethylhexyl Acrylate/Methyl Methacrylate/Butyl Acrylate/Acrylic Acid/ Methacrylic Acid copolymer (INCI name: Acrylates/Ethylhexyl Acrylate Copolymer, such as Daitosol 5000SJ sold by the company Daito Kasei Kogyo), Acrylic/Acrylates Copolymer (INCI name: Acrylates Copolymer, such as Daitosol 5000AD sold by the company Daito Kasei Kogyo), or Acrylic copolymers and Acrylates Copolymers (such as VINYSOL 2140 sold by the company Daido Chemical, Aculyn^{™} 33 sold by the company Dow Chemical, Luvimer^{®} MAE sold by the company BASF, or Balance^{®} CR sold by the company Akzo Nobel).

In other embodiments, one of the at least two latex polymers may be chosen from polyurethane latex polymers, such as aqueous polyurethane dispersions comprising the reaction products of (i), (ii), and/or (iii), defined below.

Reaction product (i) may be any prepolymer according to the formula: 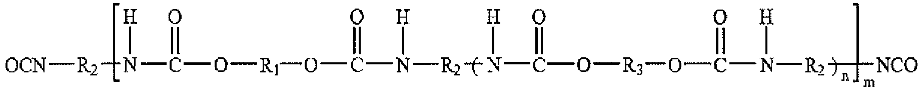 wherein R₁ is chosen from bivalent radicals of a dihydroxyl functional compound, R₂ is chosen from hydrocarbon radicals of an aliphatic or cycloaliphatic polyisocyanate, R₃ is chosen from radicals of a low molecular weight diol, optionally substituted with ionic groups, n ranges from 0 to 5, and m is greater than 1.

Suitable dihydroxyl compounds for providing the bivalent radical R₁ include those having at least two hydroxy groups, and having number average molecular weights ranging from 700 to 16,000, such as, for example, from 750 to 5000. Non-limiting examples of the high molecular weight compounds include polyester polyols, polyether polyols, polyhydroxy polycarbonates, polyhydroxy polyacetals, polyhydroxy polyacrylates, polyhydroxy polyester amides, polyhydroxy polyalkadienes and polyhydroxy polythioethers. In various embodiments, polyester polyols, polyether polyols, or polyhydroxy polycarbonates may be chosen. Mixtures of such compounds are also within the scope of the disclosure.

In some embodiments, the polyester diol may optionally be prepared from aliphatic, cycloaliphatic, or aromatic dicarboxylic or polycarboxylic acids, or anhydrides thereof; and dihydric alcohols such as diols chosen from aliphatic, alicyclic, or aromatic diols.

In other embodiments, the aliphatic dicarboxylic or polycarboxylic acids may be chosen from succinic, fumaric, glutaric, 2,2-dimethylglutaric, adipic, itaconic, pimelic, suberic, azelaic, sebacic, maleic, malonic, 2,2-dimethylmalonic, nonanedicarboxylic, decanedicarboxylic, dodecanedioic, 1,3-cyclohexanedicarboxylic, 1,4-cyclohexanedicarboxylic, 2,5-norboranedicarboxylic, diglycolic, thiodipropionic, 2,5-naphthalenedicarboxylic, 2,6-naphthalenedicarboxylic, phthalic, terephthalic, isophthalic, oxanic, o-phthalic, tetrahydrophthalic, hexahydrophthalic, trimellitic acid, or mixtures thereof.

In various embodiments, the acid anhydrides may be chosen from o-phthalic, trimellitic, succinic acid anhydride or mixtures thereof. In another embodiment, the dicarboxylic acid may be adipic acid.

In certain embodiments, the dihydric alcohols may be chosen from, for example, ethanediol, ethylene glycol, diethylene glycol, triethylene glycol, trimethylene glycol, tetraethylene glycol, 1,2-propanediol, dipropylene glycol, tripropylene glycol, tetrapropylene glycol, 1,3-propanediol, 1,4-butanediol, 1,3-butanediol, 2,3-butanediol, 1,5-pentanediol, 1,6-hexanediol, 2,2-dimethyl-1,3-propanediol, 1,4-dihydroxycyclohexane, 1,4-dimethylolcyclohexane, cyclohexanedimethanol, 1,8-octanediol, 1,10-decanediol, 1,12-dodecanediol, neopentyl glycol, or mixtures thereof. In other embodiments, the cycloaliphatic and/or aromatic dihydroxyl compounds may also be suitable as the dihydric alcohol(s) for the preparation of the polyester polyol(s).

In certain embodiments, the polyester diols may be chosen from homopolymers or copolymers of lactones, which are, in at least certain embodiments, obtained by addition reactions of lactones or lactone mixtures, such as butyrolactone, ε-caprolactone and/or methyl-ε-caprolactone with the appropriate polyfunctional, for example difunctional, starter molecules such as, for example, the dihydric alcohols mentioned above. In some embodiments, the corresponding polymers of ε-caprolactone may be chosen.

In certain embodiments, the polyester polyol, for example polyester diol, radical R₁, may be obtained by polycondensation of dicarboxylic acids, such as adipic acid, with polyols, for example diols, such as hexanediol, neopentyl glycol, or mixtures thereof.

In certain embodiments, the polycarbonates containing hydroxyl groups comprise those known per se, such as the products obtained by reacting diols, such as (1,3)-propanediol, (1,4)-butanediol and/or (1,6)-hexanediol, diethylene glycol, triethylene glycol, or tetraethylene glycol with diaryl carbonates, for example diphenyl carbonate or phosgene.

In certain embodiments, optional polyether polyols may be obtained in any known manner by reacting starting compounds which contain reactive hydrogen atoms with alkylene oxides, such as ethylene oxide; propylene oxide; butylene oxide; styrene oxide; tetrahydrofuran; epichlorohydrin, or mixtures thereof. In certain embodiments, the polyethers do not contain more than 10% by weight of ethylene oxide units. In other embodiments, polyethers obtained without addition of ethylene oxide may be chosen.

In other embodiments, polyethers modified with vinyl polymers may be chosen. Products of this type can be obtained by polymerization, for example, of styrene and acrylonitrile in the presence of polyethers, for example as described in U.S. Patent Nos. 3,383,351; 3,304,273; 3,523,095; 3,110,695; and German patent 1 152 536.

In certain embodiments, the polythioethers may be chosen from condensation products obtained from thiodiglycol per se and/or with other glycols, dicarboxylic acids, formaldehyde, aminocarboxylic acids, and/or amino alcohols. In other embodiments, the products obtained are either mixed polythioethers, polythioether esters, or polythioether ester amides, depending on the co-components.

In certain embodiments, the polyacetals may be chosen from compounds which can be prepared from aldehydes, such as formaldehyde, and from glycols, such as diethylene glycol, triethylene glycol, ethoxylated 4,4'-(dihydroxy)diphenyl-dimethylmethane, or (1,6)-hexanediol. Polyacetals according to various non-limiting embodiments of the disclosure can also be prepared by polymerization of cyclic acetals.

In certain embodiments, optional polyhydroxy polyesteramides and polyamines include, for example, the mainly linear condensation products obtained from saturated or unsaturated, polybasic carboxylic acids or anhydrides thereof; from saturated or unsaturated, polyvalent amino alcohols; from diamines; from polyamines; or mixtures thereof.

In certain embodiments, optional monomers for the production of polyacrylates having hydroxyl functionality include acrylic acid, methacrylic acid, crotonic acid, maleic anhydride, 2-hydroxyethyl acrylate, 2-hydroxyethyl methacrylate, 2-hydroxypropyl acrylate, 2-hydroxypropyl methacrylate, 3-hydroxypropyl acrylate, 3-hydroxypropyl methacrylate, glycidyl acrylate, glycidyl methacrylate, 2-isocyanatoethyl acrylate, or 2-isocyanatoethyl methacrylate.

In certain embodiments, mixtures of dihydroxy compounds may be chosen.

In various embodiments, optional polyisocyanates for providing the hydrocarbon-based radical R₂ include, for example, organic diisocyanates having a molecular weight ranging from 100 to 1500, from 112 to 1000, or from 140 to 400.

In other embodiments, optional diisocyanates are chosen from the general formula R₂(NCO)₂, in which R₂ represents a divalent aliphatic hydrocarbon group comprising from 4 to 18 carbon atoms, a divalent cycloaliphatic hydrocarbon group comprising from 5 to 15 carbon atoms, a divalent araliphatic hydrocarbon group comprising from 7 to 15 carbon atoms, or a divalent aromatic hydrocarbon group comprising from 6 to 15 carbon atoms. Examples of the organic diisocyanates include tetramethylene diisocyanate, 1,6-hexamethylene diisocyanate, dodecamethylene diisocyanate, cyclohexane-1,3-diisocyanate and cyclohexane-1,4-diisocyanate, 1-isocyanato-3-isocyanatomethyl-3,5,5-trimethylcyclohexane (isophorone diisocyanate or IPDI), bis(4-isocyanatocyclohexyl)-methane, 1,3-bis(isocyanatomethyl)cyclohexane, 1,4-bis(isocyanatomethyl)cyclohexane, bis(4-isocyanato-3-methylcyclohexyl)methane, or mixtures thereof.

In certain embodiments, the diisocyanates are chosen from aliphatic or cycloaliphatic diisocyanates, for example, 1,6-hexamethylene diisocyanate, isophorone diisocyanate, dicyclohexylmethane diisocyanate, or mixtures thereof.

In some embodiments, the use of diols, for example low molecular weight diols, R₃, may allow a stiffening of the polymer chain. The expression "low molecular weight diols" means diols having a molecular weight ranging from 50 to 800, such as 60 to 700, or 62 to 200. They may, in various embodiments, contain aliphatic, alicyclic, or aromatic groups. In certain embodiments, the compounds contain only aliphatic groups. In other embodiments, the diols may have up to 20 carbon atoms, and may be chosen, for example, from ethylene glycol, diethylene glycol, propane-1,2-diol, propane-1,3-diol, butane-1,4-diol, 1,3-butylene glycol, neopentyl glycol, butylethylpropanediol, cyclohexanediol, 1,4-cyclohexanedimethanol, hexane-1,6-diol, bisphenol A (2,2-bis(4-hydroxyphenyl)propane), hydrogenated bisphenol A (2,2-bis(4-hydroxycyclohexyl)propane), or mixtures thereof.

In other embodiments, the low molecular weight diols may contain ionic or potentially ionic groups. Suitable low molecular weight diols containing ionic or potentially ionic groups may be chosen from those disclosed in U.S. Patent No. 3,412,054. In various embodiments, compounds may be chosen from dimethylolbutanoic acid (DMBA), dimethylolpropionic acid (DMPA), or carboxyl-containing caprolactone polyester diol. If low molecular weight diols containing ionic or potentially ionic groups are chosen, they may be used in an amount such that less than 0.30 meq of -COOH is present per gram of polyurethane in the polyurethane dispersion. In certain embodiments, the low molecular weight diols containing ionic or potentially ionic groups are not used.

Reaction product (ii) may be chosen from at least one chain extender according to the formula:

H₂N - R4 - NH₂

wherein R4 is chosen from alkylene or alkylene oxide radicals, said radicals not being substituted with ionic or potentially ionic groups.

Reaction product (ii) may optionally be chosen from alkylene diamines,
such as hydrazine, ethylenediamine, propylenediamine, 1,4-butylenediamine or piperazine; and alkylene oxide diamines such as dipropylamine diethylene glycol (such as DPA-DEG sold by the company Tomah Products), 2-methyl-1,5-pentanediamine (such as Dytec A sold by the company DuPont), hexanediamine, isophoronediamine, and 4,4-methylenedi(cyclohexylamine), and the DPA-series of ether amines available from the company Tomah Products, including dipropylamine propylene glycol, dipropylamine dipropylene glycol, dipropylamine tripropylene glycol, dipropylamine polypropylene glycol), dipropylamine ethylene glycol, dipropylamine polyethylene glycol), dipropylamine 1,3-propanediol, dipropylamine 2-methyl-1,3-propanediol, dipropylamine 1,4-butanediol, dipropylamine 1,3-butanediol, dipropylamine 1,6-hexanediol, dipropylamine cyclohexane-1,4-dimethanol, or mixtures thereof.

Reaction product (iii) may be chosen from at least one chain extender according to the formula:

H₂N - R5 - NH₂

wherein R5 is chosen from alkylene radicals substituted with ionic or potentially ionic groups. In certain exemplary embodiments, the compounds may have an ionic or potentially ionic group and two isocyanate-reactive groups.

As used herein, ionic or potentially ionic groups may include groups comprising ternary or quaternary ammonium groups, groups convertible into such groups, carboxyl groups, carboxylate groups, sulfonic acid groups, and sulphonate groups. At least partial conversion of the groups convertible into salt groups of the type mentioned may take place before or during the mixing with water. Specific compounds include diaminosulphonates, for example the sodium salt of N-(2-aminoethyl)-2-aminoethanesulfonic acid (AAS) or the sodium salt of N-(2-aminoethyl)-2- aminopropionic acid.

In certain embodiments, R5 represents an alkylene radical substituted with sulfonic acid or sulfonate groups. By way of example only, the reaction product (iii) is chosen from sodium salts of N-(2-aminoethyl)-2-aminoethanesulfonic acid.

In certain embodiments, such latexes include, but are not limited to, aqueous polyurethane dispersions comprising a reaction product of a prepolymer comprising a dihydroxyl compound, a polyisocyanate, a low molecular weight diol, at least two diamine compounds, or wherein the composition is substantially free of triethanolamine stearate such as, those sold under the tradename Baycusan^{®} by Bayer such as, Baycusan^{®} C1000 (INCI name: Polyurethane-34), Baycusan^{®} C1001 (INCI name: Polyurethane-34), Baycusan^{®} C1003 (INCI name: Polyurethane-32), Baycusan^{®} C1004 (INCI name: Polyurethane-35) and Baycusan^{®} C1008 (INCI name: Polyurethane-48). In various embodiments, polyurethane latexes may be chosen from, but are not limited to, aqueous polyurethane dispersion of Isophthalic Acid/Adipic Acid/Hexylene Glycol/Neopentyl glycol/Dimethylolpropanoic Acid/lsophorone Diisocyanate copolymer (INCI name: Polyurethane-1, such as Luviset^{®} P.U.R sold by the company BASF), aliphatic polyurethane and aliphatic polyester polyurethane (INCI name: Polycarbamyl Polyglycon Ester such as the Neorez^{®} series sold by the company DSM, including Neorez^{®} R989).

In certain embodiments, the at least two latex polymers may be chosen from polyacrylic latex, polyacrylate latex, polystyrene latex, polyester latex, polyamide latex, polyurea latex, polyurethane latex, epoxy resin latex, cellulose-acrylate latex, or their copolymers.

In various embodiments according to the disclosure, it may be possible to choose a polymer that comprises both acrylate and polyurethane parts at the molecular level.

In one embodiment, the compositions of the present disclosure contain two latex polymers comprising acrylates copolymer as sold under the tradename LUVIFLEX SOFT by BASF and polyurethane-34 as sold under the tradename Baycusan^{®} by Bayer Material Science.

As described herein, certain embodiments according to the disclosure may comprise at least two latex polymers chosen from acrylate and polyurethane polymers, wherein at least one of the latex polymers is a film-forming polymer, with the proviso that when the first latex polymer is chosen from acrylate polymers, the second latex polymer is chosen from polyurethane polymers; and when the first latex polymer is chosen from polyurethane polymers, the second latex polymer is chosen from acrylate polymers.

In certain embodiments, each of the latex polymers is present in an amount ranging from 0.001% to 15% by weight, from 0.05% to 10% by weight, from 0.1% to 7.5% by weight, from 0.25% to 5% by weight, from 0.5% to 2.5% by weight, or from 0.5% to 1.5% by weight, relative to the weight of the composition, including all ranges and subranges therebetween.

In other embodiments, each of the latex polymers is present in an amount ranging from 1% to 15% by weight, from 1% to 12% by weight, from 1.2% to 12% by weight, from 1.5% to 10% by weight, or less than 10% by weight, relative to the weight of the composition, including all ranges and subranges therebetween.

In certain embodiments, the latex polymers are present in a combined amount ranging from 0.1% to 30% by weight, from 0.25% to 20% by weight, or from 0.3 % to 15% by weight, or from 0.4 % to 10% by weight, or from 0.5% to 5% by weight, relative to the weight of the composition, including all ranges and subranges therebetween.

In other embodiments, the combined amount of latex polymers may be 0.1%, 0.5%, 0.6%, 0.75, 0.9%, 1%, 1.5%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, or 30%, by weight, relative to the weight of the composition.

In further embodiments, the combined amount of latex polymers ranges up to 30%, up to 29%, up to 28%, up to 27%, up to 26%, up to 25%, up to 24%, up to 23%, up to 22%, up to 21%, up to 20%, up to 19%, up to 18%, up to 17%, up to 16%, up to 15%, up to 14%, up to 13%, up to 12%, up to 11%, up to 10%, up to 10%, up to 9%, up to 8%, up to 7%, up to 6%, up to 5%, up to 4%, up to 3%, up to 2%, or up to 1%, each by weight, relative to the weight of the composition.

In certain embodiments, the combined amount of latex polymers is less than 10% by weight or less than 5% by weight, relative to the weight of the composition.

In certain embodiments, the weight ratio of the at least two latex polymers, e.g. polymer A to polymer B, may range from 10:1 to 1:10, from 9:1 to 1:9, from 8:1 to 1:8, from 7:1 to 1:7, from 6:1 to 1:6, from 5:1 to 1:5, from 4:1 to 1:4, from 3:1 to 1:3, or from 2:1 to 1:2, including all ranges and subranges therebetween.

In other embodiments, the weight ratio of polymer A to polymer B is 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, or 1:10.

In certain embodiments, the at least two latex polymers composition of the present invention comprise at least one acrylate latex polymer and at least one polyurethane latex polymer and mayrange from 10:1 to 1:10, from 9:1 to 1:9, from 8:1 to 1:8, from 7:1 to 1:7, from 6:1 to 1:6, from 5:1 to 1:5, from 4:1 to 1:4, from 3:1 to 1:3, or from 2:1 to 1:2.

In some embodiments, the weight ratio of the acrylate latex polymer to the polyurethane latex polymer may range from 1 to 1:10, from 1 to 1:9, from 1 to 1:8, from 1 to 1:7, from 1 to 1:6, from 1 to 1:5, from 1 to 1:4, from 1 to 1:3, or from 1 to 1:2, including all ranges and subranges therebetween.

In some embodiments, the weight ratio of the acrylate latex polymer to the polyurethane latex polymer may range from 10:1 to 2:1, from 9:1 to 3:1, from 8:1 to 4:1, from 7:1 to 5:1, or from 6:1 to 5:1, including all ranges and subranges therebetween.

In other embodiments, the weight ratio of the acrylate latex polymer to the polyurethane latex polymer is 1, 1.1, 1.2, 1.3, 1.4, 1.5, 1.6, 1.7, 1.8, 1.9, 2, 2.5, 3, 3.5, 4, 4.5, 5, 5.5, 6, 6.5, 7, 7.5, 8, 8.5, 9, 9.5, or 10.

In certain embodiments, when polymer A is chosen from latex polymers having a Young's modulus ranging from 0.1 MPa to 10 MPa and a strain, under stress at 0.5 MPa, of at least 1%, and polymer B is chosen from latex polymers having a Young's modulus ranging from 10 MPa to 6 GPa and a strain, under stress at 0.5 MPa, of less than 5%, different weight ratios of polymer A to polymer B may be chosen to correspond to different hair styling applications.

In other embodiments, various weight ratios of the two latex polymers provide different levels of style hold on hair ranging from a high level of style hold to a medium to high level of style hold to a light to a medium level of style hold or to a light hold.

### AMINOFUNCTIONAL SILICONE

The compositions according to the disclosed embodiments comprise at least one aminofunctional silicone chosen from amodimethicone/morpholinomethyl silsesquioxane copolymer, PEG-40/PPG-8 methylaminopropyl/hydroxypropyl dimethicone copolymer, trideceth-9 PG-amodimethicone, or mixtures thereof.

For example, Trideceth-9 PG-Amodimethicone is sold under the tradename Silcare^{®} Silicone SEA by the company Clariant, -PEG-40/PPG-8 methylaminopropyl/hydroxypropyl dimethicone copolymer is sold under the tradename Silsoft^{®} A+ from Momentive, and amodimethicone/morpholinomethyl silsesquioxane copolymer is sold under the tradename Belsil^{®} ADM 8301 E from Wacker.

The at least one aminofunctional silicone is present in an amount ranging from 0.25% to 3%, and preferably from 0.25% to 2.5% by weight, relative to the total weight of the composition, including all ranges and subranges therebetween.

In various embodiments, the total amount of amino functional silicone is 0.3%, 0.4%, 0.5%, 0.6%, 0.7%, 0.8%, 0.9%, 1%, 1.25%, 1.5%, 2%, 2.5% or 3% by weight, based on the total weight of the composition.

### THICKENING AGENT

The compositions according to the present disclosure comprise at least one thickening agent chosen from optionally crosslinked and/or neutralized 2-acrylamido-2-methylpropanesulfonic acid polymers and copolymers, for instance poly(2-acrylamido-2-methylpropanesulfonic acid) such as those sold under the tradename Hostacerin^{®} AMPS (INCI name: ammonium polyacryldimethyltauramide) by the company Clariant, crosslinked anionic copolymers of acrylamide and of AMPS, e.g. in the form of a water-in-oil emulsion, such as those sold under the tradename Sepigel^{™} 305 (INCI name: Polyacrylamide/C13-14 Isoparaffin/Laureth-7) and under the tradename Simugel^{™} 600 (CTFA name: Acrylamide/Sodium acryloyldimethyltaurate copolymer/Isohexadecane/Polysorbate 80) by the company Seppic.

Thickening agents are generally used to modify the viscosity and/or rheology of the composition. As used herein, the term "thickening agent" means compounds which, by their presence, increase the viscosity of the composition into which they are introduced by at least 20 cps, such as by at least 50 cps, at 25°C and at a shear rate of 1 s-1. The viscosity may be measured using a cone/plate viscometer, a Haake R600 rheometer, or the like. Thickening agents may also sometimes be referred to as gellifying agents and/or viscosity modifying and/or rheology-modifying agents.

The at least one thickening agent is present in the composition of the present invention in an amount ranging from 0.05% to 10% by weight, from 0.1% to 8% by weight, from 0.2% to 7% by weight, from 0.25% to 6%, and from 0.3% to 5% by weight, relative to the total weight of the composition, including all ranges and subranges therebetween.

In various embodiments, the total amount of the at least one thickening agent is 0.05%, 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1%, 1.25%, 1.5%, 1.75%, 2%, 2.25%, 2.5% 2.75%, 3%, 3.25%, 3.5%, 3.75%, 4%, 4.25%, 4.5%, 4.75%, or 5%, by weight, based on the total weight of the composition.

### NONIONIC SURFACTANT

The compositions according to the present disclosure comprise at least one nonionic surfactant chosen glyceryl esters, fatty alcohols, alkoxylated alcohols and lanolin, alkylpolyglucosides, or mixtures thereof. Nonionic surfactants can also be employed as emulsifying agents in the compositions of the present invention or in the emulsions comprising the at least one thickening agent chosen from emulsions of crosslinked anionic copolymers of acrylamide/nonionic surfactants, or emulsions of AMPS/nonionic surfactants.

### Glyceryl Esters

The glyceryl esters in the compositions of the present invention include, but are not limited to, glyceryl monoesters, such as glyceryl monoesters of C16-C22 saturated, unsaturated and branched chain fatty acids such as glyceryl oleate, glyceryl monostearate, glyceryl monoisostearate, glyceryl monopalmitate, glyceryl monobehenate, and mixtures thereof.

The glyceryl esters in the compositions of the present invention may also be reffered to as nonionic co-emulsifiers.

In one embodiment, the glyceryl ester is chosen from glyceryl oleate, glyceryl monostearate (glyceryl stearate), glyceryl monoisostearate, glyceryl monopalmitate, glyceryl monobehenate, and mixtures thereof.

In one embodiment, the glyceryl ester is chosen from glyceryl stearate.

### Fatty alcohols

The fatty alcohols that may be used in the composition of the disclosure include, but are not limited to, non-alkoxylated, saturated or unsaturated, linear or branched, and have from 6 to 60 carbon atoms, such as from 8 to 30 carbon atoms.

The fatty alcohols of the present disclosure are chosen from solid and liquid fatty alcohols.

The saturated liquid fatty alcohols can be branched. They can optionally comprise, in their structure, at least one aromatic or non-aromatic ring. They can be acyclic.

The unsaturated liquid fatty alcohols exhibit, in their structure, at least one double or triple bond and preferably one or more double bonds. When several double bonds are present, there are preferably 2 or 3 of them and they can be conjugated or unconjugated. These unsaturated fatty alcohols can be linear or branched. They can optionally comprise, in their structure, at least one aromatic or non-aromatic ring. They can be acyclic. Among the liquid unsaturated fatty alcohols, oleyl alcohol, linoleyl alcohol, linolenyl alcohol and undecylenyl alcohol may be mentioned.

Liquid fatty alcohols may be selected, for example, from octyldodecanol, 2-butyloctanol, 2-hexyldecanol, 2-undecylpentadecanol, oleyl alcohol or linoleyl alcohol, isostearyl alcohol, and mixtures thereof.

Solid fatty alcohols may be crystalline, amorphous or pasty. The solid fatty alcohols of the present invention are solid at room temperature (25 degrees centigrade) and at atmospheric pressure (1 atm) and are insoluble in water (i.e. they have a solubility in water of less than 1% by weight and preferably less than 0.5% by weight, at 25°C and 1 atm) and are soluble, under the same temperature and pressure conditions, in at least one organic solvent (for example ethanol, chloroform, benzene or liquid petroleum jelly) to at least 1% by weight.

In an embodiment, the solid fatty alcohols preferably have a melting point of greater than or equal to 28°C and have a viscosity, at a temperature of 40 °C and at a shear rate of 1 s⁻¹, of greater than or equal to 1 Pa.s.

In an embodiment, the melting point of the fatty alcohols ranges from 30°C to 250 °C, such as from 32 °C to 150°C or such as from 35°C to 150°C.

The melting points may be measured by DSC or on a Kofler bench. The melting point may be measured by differential calorimetric analysis (DSC) with a temperature rise of 10°C per minute. The melting point is then the temperature corresponding to the top of the melting endotherm peak obtained during the measurement.

The viscosity measurements may be taken at a temperature of 40 °C using an RS600 rheometer from Thermoelectron.

The solid fatty alcohols of the present invention are chosen from saturated or unsaturated, linear or branched, preferably linear and saturated, (mono) alcohols comprising from 6 to 60 carbon atoms, such as from 10 to 50 carbon atoms, or such as from 12 to 24 carbon atoms.

The solid fatty alcohols preferably have the structure of formula: R-OH in which R especially denotes a C6-C60, for example, C8-C60, preferably C10-C50 or even C12-C30 alkyl group, R possibly being substituted with one or more hydroxyl groups, R possibly being branched. The solid fatty alcohols of the invention may be non- oxyalkylenated and/or non-glycerolated. These fatty alcohols may be constituents of animal or plant waxes.

The solid fatty alcohol may represent a mixture of fatty alcohols, which means that several species of fatty alcohol may coexist, in the form of a mixture, in a commercial product. One example of such a commercial product is cetearyl alcohol, a mixture of cetyl alcohol and stearyl alcohol, commercially available under the trade name of LANETTE-O from the company BASF. Cetyl alcohol may also be commercially available under the tradename of LANETTE 16 from the company BASF.

In an embodiment, the solid fatty alcohols of the present invention may be chosen from myristyl alcohol, cetyl alcohol, stearyl alcohol, cetearyl alcohol, and mixtures thereof, octyldodecanol, 2-butyloctanol, 2-hexyldecanol, 2-undecylpentadecanol, oleic alcohol, linoleic alcohol, behenyl alcohol, and mixtures thereof.

Other suitable examples of the solid fatty alcohol of the present invention include branched solid fatty alcohols chosen from 2-dodecylhexadecanol, 2-tetradecyl-1-octadecanol, 2-tetradecyl-1-eicosanol, 2-hexadecyl-1-octadecanol and 2-hexadecyl-1-eicosanol, and mixtures thereof.

In embodiment of the present invention, the fatty alcohol is chosen from cetyl alcohol.

In embodiment of the present invention, the fatty alcohol is chosen from cetearyl alcohol.

In embodiment of the present invention, the fatty alcohol comprises cetyl alcohol and cetearyl alcohol.

In embodiment of the present invention, the fatty alcohol comprises cetyl alcohol and stearyl alcohol.

In embodiment of the present invention, the fatty alcohol is chosen from cetyl alcohol, stearyl alcohol, cetearyl alcohol, and mixtures thereof

In an embodiment, the fatty alcohols of the present invention are chosen from liquid fatty alcohol, solid fatty alcohols, and mixtures thereof.

### Alkoxylated alcohols and lanolin

"Alkoxylated fatty alcohol" as used herein means a compound having at least one fatty portion (8 carbon atoms or more) and at least one alkoxylated portion (-(CH2)nO-, where n is an integer from 1 to 5, preferably 2 to 3). According to particularly preferred embodiments, the alkoxylated fatty alcohols of the present invention can be used as non-ionic surfactants, if desired. In this regard, the alkoxylated fatty alcohols of the present invention preferably have an HLB (hydrophilic-lipophilic balance) value from 1-20, including all ranges and subranges therebetween, with HLB values ranging from 1 to 5 (particularly 3 to 5) or from 15-20 (particularly 16 to 18) being most preferred. Preferably, the alkoxylated fatty alcohol is chosen from ethoxylated fatty alcohols, propoxylated fatty alcohols, and mixtures thereof.

Preferably, the alkoxylated fatty alcohol can be chosen from di-alkyl, tri-alkyl- and combinations of di-alkyl and tri-alkyl substituted ethoxylated polymers. They can also be chosen from mono-alkyl, di-alkyl, tri-alkyl, tetra-alkyl substituted alkyl ethoxylated polymers and all combinations thereof. The alkyl group can be saturated or unsaturated, branched or linear and contain a number of carbon atoms preferably from 12 carbon atoms to 50 carbon atoms, including all ranges and subranges therebetween, for example, 20 to 40 carbon atoms, 22 to 24 carbon atoms, 30 to 50 carbon atoms, and 40 to 60 carbon atoms. Most preferably, the fatty portion contains a mixture of compounds of varying carbon atoms such as, for example, C20-C40 compounds, C22-C24 compounds, C30-050 compounds, and C40-C60 compounds.

Preferably, the alkoxylated portion of the alkoxylated fatty alcohols of the present invention contain 2 or more alkoxylation units, preferably from 10 to 200 alkoxylation units, preferably from 20 to 150 alkoxylation units, and preferably from 25 to 100 alkoxylation units, including all ranges and subranges therebetween. Also preferably, the alkoxylation units contain 2 carbon atoms (ethoxylation units) and/or 3 carbon atoms (propoxylation units).

The amount of alkoxylation can also be determined by the percent by weight of the alkoxylated portion with respect to the total weight of the compound. Suitable weight percentages of the alkoxylated portion with respect to the total weight of the compound include, but are not limited to, 10 percent to 95 percent, preferably 20 percent to 90 percent, including all ranges and subranges therebetween with 75 percent to 90 percent (particularly 80 percent to 90 percent) or 20 percent to 50 percent being preferred.

Preferably, the alkoxylated fatty alcohols of the present invention have a number average molecular weight (Mn) greater than 500, preferably from 500 to 5,000, including all ranges and subranges therebetween such as, for example, Mn of 500 to 1250 or an Mn of 2,000 to 5,000.

Suitable examples of alkoxylated fatty alcohols include: laureth-3, laureth-7, laureth-9, laureth-12, laureth-23 ceteth-10, steareth-10, steareth-2, steareth-100, beheneth-5, beheneth-5, beheneth-10, oleth-10, Pareth alcohols, trideceth-10, trideceth-12, C12-13 pareth-3, C12-13 pareth-23, C11-15 pareth-7, PEG hydrogenated castore oil, PEG-75 lanolin, polysorbate-80, polysobate-20, PPG-5 ceteth-20, PEG-55 Propylene Glycol Oleate, glycereth-26 (PEG-26 Glyceryl Ether), PEG 120 methyl glucose dioleate, PEG 120 methyl glucose trioleate, PEG 150 pentaerythrityl tetrastearate, and mixtures thereof.

### Alkylpolyglucosides

The alkyl(poly)glucoside (alkylpolyglycoside) is represented especially by the following general formula:

R₁O-(R₂O)ₜ-(G)ᵥ

wherein:
- R₁ represents a linear or branched alkyl or alkenyl radical comprising 6 to 24 carbon atoms and especially 8 to 18 carbon atoms, or an alkylphenyl radical whose linear or branched alkyl radical comprises 6 to 24 carbon atoms and especially 8 to 18 carbon atoms;
- R₂ represents an alkylene radical comprising 2 to 4 carbon atoms,
- G represents a sugar unit comprising 5 to 6 carbon atoms,
- t denotes a value ranging from 0 to 10 and preferably 0 to 4,
- v denotes a value ranging from 1 to 15 and preferably 1 to 4.

Preferably, the alkylpolyglycoside surfactants are compounds of the formula described above in which:
- R₁ denotes a linear or branched, saturated or unsaturated alkyl radical comprising from 8 to 18 carbon atoms,
- R₂ represents an alkylene radical comprising 2 to 4 carbon atoms,
- t denotes a value ranging from 0 to 3 and preferably equal to 0,
- G denotes glucose, fructose or galactose, preferably glucose;
- the degree of polymerization, i.e. the value of v, possibly ranging from 1 to 15 and preferably from 1 to 4; the mean degree of polymerization more particularly being between 1 and 2.

The glucoside bonds between the sugar units are generally of 1-6 or 1-4 type and preferably of 1-4 type. Preferably, the alkyl(poly)glycoside surfactant is an alkyl(poly)glucoside surfactant. C8/C16 alkyl(poly)glycosides 1,4, and especially decyl glucosides and caprylyl/capryl glucosides, are most particularly preferred.

Among the commercial products, mention may be made of the products sold by the company COGNIS under the names PLANTAREN^{®} (600 CS/U, 1200 and 2000) or PLANTACARE^{®} (818, 1200 and 2000); the products sold by the company SEPPIC under the names ORAMIX CG 110 and ORAMIX NS 10; the products sold by the company BASF under the name LUTENSOL GD 70, or else the products sold by the company CHEM Y under the name AG10 LK.

Preferably, use is made of C8/C16-alkyl(poly)glucosides 1,4, especially as an aqueous 53% solution, such as those sold by Cognis under the reference Plantacare^{®} 818 UP.

### In an embodiment, the alkylpolyglucosides are chosen from decyl glucoside, cetearyl glucoside, stearyl glucoside, lauryl glucoside, coco-glucoside, and mixtures thereof.

In an embodiment, the alkylpolyglucosides are chosen from cetearyl glucoside, decyl glucoside, and mixtures thereof.

In the present invention, the at least one nonionic surfactant may be employed in the composition of the present invention in an amount of from 0.05% to 8% by weight, such as from 0.075% to 7% by weight, from 0.1% to 6% by weight, from 0.2% to 5% by weight, or from 0.3% to 5% by weight of active material, based on the total weight of the composition, including all ranges and subranges therebetween.

In various embodiments, the total amount of the at least one glyceryl ester is 0.05%, 0.075%, 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.86%, 0.9%, 0.95%, 1%, 1.25%, 1.5%, 1.75%, 2%, 2.25%, 2.5%, 2.75%, 3%, 3.25%, 3.5%, 3.75%, 4%,4.25%, 4.5%, 4.75%, 5%, 5.25%, 5.5%, 5.75%, 6% or 6.5%, 7%, 7.5%, or 8% by weight of active material, based on the total weight of the composition.

### NON-LATEX NONIONIC FILM FORMING POLYMER

The at least one non-latex nonionic film forming polymer which can be used according to the present disclosure is chosen from
- polyalkyloxazolines;
- vinyl acetate homopolymers;
- vinyl acetate copolymers, for instance copolymers of vinyl acetate and of acrylic ester, copolymers of vinyl acetate and of ethylene, or copolymers of vinyl acetate and of maleic ester, for example of dibutyl maleate;
- homopolymers and copolymers of acrylic esters, such as for example, copolymers of alkyl acrylates and alkyl methacrylates2;
- copolymers of acrylonitrile and a nonionic monomer, chosen, for example, from butadiene and alkyl (meth)acrylates;
- styrene homopolymers;
- styrene copolymers, for instance copolymers of styrene and of an alkyl (meth)acrylate; copolymers of styrene, of alkyl methacrylate and of alkyl acrylate; copolymers of styrene and of butadiene; or copolymers of styrene, of butadiene and of vinylpyridine;
- polyamides;
- vinylpyrrolidone homopolymers;
- copolymer of vinylpyrrolidone and vinyl acetate monomers;
- vinyllactam homopolymers such as vinylpyrrolidone homopolymers, and such as polyvinylcaprolactam; and
- vinyllactam copolymers such as a poly(vinylpyrrolidone/vinyllactam) copolymer, poly(vinylpyrrolidone/vinyl acetate) copolymers; and poly(vinylpyrrolidone/vinyl acetate/vinyl propionate) terpolymers.

Suitable examples are: vinylpyrrolidone homopolymers; copolymers of vinylpyrrolidone and of vinyl acetate; polyalkyloxazolines, such as the polyethyloxazolines provided by the company Polymer Chemistry Innovations under the names AQUAZOL HP, and AQUAZOL HVIS; vinyl acetate homopolymers, such as the product provided under the name UCAR^{™} 130 Latex Resin by the company Dow Chemical or the product provided under the name Ultrapure Polymer 2041-R 012 by the company Ultra Chemical, Inc.; copolymers of vinyl acetate and of acrylic ester, such as the product provided under the name RHODOPAS AD 310 from Rhone-Poulenc; copolymers of vinyl acetate and of ethylene, such as the product provided under the name DERMACRYL LOR by the company Akzo Nobel; copolymers of vinyl acetate and of maleic ester, for example of dibutyl maleate, such as the product provided under the nameAPPRETAN MB Extra by the company Clariant; copolymers of polyethylene and of maleic anhydride; alkyl acrylate homopolymers and alkyl methacrylate homopolymers, such as the product provided under the name MICROPEARL RQ 750 by the company Matsumoto or the product provided under the name LUHYDRAN A 848 5 by the company BASF; acrylic ester copolymers, such as, for example, copolymers of alkyl acrylates and of alkyl methacrylates, such as the product provided by the company Dow Chemical under the name PRIMAL AC-261 K and the product provided by Evonik under the name EUDRAGIT NE 30 D, by the company BASF under the names ACRONAL 601, LUHYDRAN R 8833 or 8845, or by the company Clariant under the names APPRETAN N 9213 or N9212; copolymers of acrylonitrile and of a non-ionic monomer chosen, for example, from butadiene and alkyl (meth)acrylates; mention may be made of the products provided under the names Nipol LX 531 B by the company Nippon Zeon or those provided under the name CJ 0601 B by the company Rohm and Haas; polyurethanes, such as the products provided under the names ACRYSOL RM 1020 or ACRYSOL RM 2020 by the company Dow Chemical or the products URAFLEX XP 401 UZ or URAFLEX XP 402 UZ by the company DSM Resins; copolymers of alkyl acrylate and of urethane, such as the product 8538-33 by the company National Starch; polyamides, such as the product ESTAPOR LO 11 provided by the company Rhone-Poulenc; and chemically modified or unmodified non-ionic guar gums.

The unmodified non-ionic guar gums are, for example, the products sold under the name VIDOGUM GH by the company Unipectine and under the name JAGUAR S by the company Rhodia. The modified non-ionic guar gums, which can be used according to the invention, are preferably modified by C1-C6 hydroxyalkyl groups. Mention may be made, by way of example, of the hydroxymethyl, hydroxyethyl, hydroxypropyl, and hydroxybutyl groups. These guar gums are well known in the state of the art and can, for example, be prepared by reacting the corresponding alkene oxides, such as, for example, propylene oxides, with guar gum, so as to obtain a guar gum modified by hydroxypropyl groups.

Other nonionic film forming polymers may be chosen from non-ionic guar gums optionally modified by hydroxyalkyl groups are, for example, sold under the trade names JAGUAR HP8, JAGUAR HP60, JAGUAR HP120, and JAGUAR HP 105 by the company Rhodia or under the name GALACTOSOL 4H4FD2 by the company Ashland Specialty Ingredients.

Preferred nonionic film forming polymers of the present disclosure are chosen from vinylpyrrolidone homopolymers and copolymers of vinylpyrrolidone and of vinyl acetate. Vinylpyrrolidone homopolymers (INCI name: polyvinylpyrrolidone) are commercially available from Ashland Specialty ingredients under the tradename PVP K. Copolymers of vinylpyrrolidone and of vinyl acetate (INCI name: VP/VA copolymer) are commercially available from BASF under the tradename LUVISKOL VA.

The at least one non-latex nonionic film forming polymer is present in the composition of the present disclosure in an amount of from 0.05% to 15% by weight, such as from 0.1% to 10% by weight, and from 0.5% to 5% by weight, including all ranges and subranges there-between, based on the total weight of the composition.

### ADDITIONAL THICKENING AGENTS/RHEOLOGY MODIFIERS

The compositions according to the disclosed embodiments may further comprise an additional thickening agent, also referred to interchangeably herein as thickener or rheology modifier. The additional thickening agent is other than the at least one thickening agent of the present invention chosen from optionally crosslinked and/or neutralized 2-acrylamido-2-methylpropanesulfonic acid polymers (AMPS) and copolymers, crosslinked anionic copolymers of acrylamide, crosslinked anionic copolymers of AMPS, emulsions of crosslinked anionic copolymers of acrylamide/nonionic surfactants, or emulsions of AMPS/nonionic surfactants.

In certain embodiments, the additional thickening agent may be chosen from those conventionally used in cosmetics, such as polymers of natural origin and synthetic polymers, for example, nonionic, anionic, cationic, amphiphilic, or amphoteric polymers, and other known rheology modifiers, such as cellulose-based thickeners. The additional thickening agents may also have film forming properties.

In certain embodiments, the thickening agents can be an anionic thickening agent.

The anionic thickening agent of the present disclosure may be chosen from acrylate- or acrylic-based polymers, carbomers, crosslinked homopolymers of acrylic acid, and crosslinked copolymers of (meth)acrylic acid and/or (C1-C6)alkyl esters.

Another example of an anionic thickening agent is a copolymer of ammonium acryloyldimethyltaurate and vinylpyrrolidone monomers, for example, Ammonium AcryloyldimethyltaurateNP Copolymer, sold under the tradename ARISTOFLEX AVC by the company Clariant.

The anionic thickening agents may be chosen from hydrophilic thickeners. As used herein, the term "hydrophilic thickener" is meant to indicate that the thickening agent is soluble or dispersible in water. Non-limiting examples of hydrophilic thickeners include homopolymers or copolymers of acrylic or methacrylic acids or the salts thereof and the esters thereof, such as those sold under the tradenames Versicol F^{®} or Versicol K^{®} by the company Allied Colloid, or under the tradename Ultrahold 8^{®} by the company Ciba-Geigy; polyacrylates and polymethacrylates such as copolymers of (meth)acrylic acid, copolymers of (meth)acrylic acid, methylacrylate and dimethyl meta-isopropenyl benzyl isocyanate of ethoxylated alcohols such as methylacrylate and dimethyl meta-isopropenyl benzyl isocyanate of ethoxylated alcohol (INCI name: Polyacrylate-3) sold under the tradename Viscophobe^{®} DB 1000 from The Dow Chemical Company, those sold under the tradenames Lubrajel and Norgel by the company Guardian, or under the tradename Hispajel by the company Hispano Chimica; and polyacrylic acids of Synthalen^{®} K type, copolymers of acrylic acid and of acrylamide sold in the form of the sodium salt thereof, such as those sold under the tradenames Reten^{®} by Hercules, sodium polymethacrylate such as those sold under the tradename Darvan^{®} 7 by the company Vanderbilt, and the sodium salts of polyhydroxycarboxylic acids such as those sold under the tradename Hydagen F^{®} by the company Henkel, polyacrylic acid/alkyl acrylate copolymers of PemulenTM type, associative polymers, for instance PEG-150/stearyl alcohol/SMDI copolymer such those as sold under the tradename ACULYN^{™} 46 by the company Rohm & Haas, steareth-100/PEG-136/HDI copolymer such as those sold under the tradename Rheolate^{®} FX 1100 by the company Elementis, and mixtures thereof.

As used herein, the term "copolymers" is intended to mean both copolymers obtained from two types of monomers and those obtained from more than two types of monomers, such as, for example, terpolymers obtained from three types of monomers. The chemical structure of the copolymers comprises at least one hydrophilic unit and at least one hydrophobic unit. The expression "hydrophobic unit" or "hydrophobic unit" is understood to mean a radical possessing a saturated or unsaturated and linear or branched hydrocarbon-based chain which comprises at least 8 carbon atoms, for example from 10 to 30 carbon atoms, as a further example from 12 to 30 carbon atoms, and as yet a further example from 18 to 30 carbon atoms.

In certain embodiments, the hydrophilic thickener may be chosen from anionic associative polymers. As used herein, the term "associative polymer" is intended to mean any polymer comprising in its structure at least one fatty chain and at least one hydrophilic portion.

In certain embodiments, the associative polymers may be chosen from polymers comprising at least one hydrophilic unit and at least one fatty-chain allyl ether unit; polymers in which the hydrophilic unit is constituted of an ethylenic unsaturated anionic monomer, such as a vinylcarboxylic acid, acrylic acid, methacrylic acid, or mixtures thereof; and polymers in which the fatty-chain allyl ether unit corresponds to the monomer of formula (I) below:

CH2 = C(R')CH2OBnR (I)

in which R' is chosen from H or CH3, B is an ethyleneoxy radical, n is zero or is chosen from an integer ranging from 1 to 100, and R is a hydrocarbon-based radical chosen from alkyl, arylalkyl, aryl, alkylaryl, or cycloalkyl radicals containing from 8 to 30 carbon atoms, from 10 to 24 carbon atoms, or from 12 to 18 carbon atoms. Exemplary and non-limiting polymers of this type are described and prepared, according to an emulsion polymerization process, in patent EP 0 216 479.

In certain embodiments, the associative anionic polymer may be chosen from anionic polymers comprising at least one hydrophilic unit of olefinic unsaturated carboxylic acid type, and at least one hydrophobic unit exclusively of (C10 C30)alkyl ester of unsaturated carboxylic acid type.

In certain embodiments, the at least one thickening agent is chosen from copolymers resulting from the polymerization of at least one monomer (a) chosen from carboxylic acids possessing α,β-ethylenically unsaturated groups or their esters, with at least one monomer (b) possessing ethylenically unsaturated groups and comprising a hydrophobic group. Such copolymers may exhibit emulsifying properties.

As used herein, the term "copolymers" is intended to mean both copolymers obtained from two types of monomers and those obtained from more than two types of monomers, such as, for example, terpolymers obtained from three types of monomers. The chemical structure of the copolymers comprises at least one hydrophilic unit and at least one hydrophobic unit. The expression "hydrophobic unit" or "hydrophobic unit" is understood to mean a radical possessing a saturated or unsaturated and linear or branched hydrocarbon-based chain which comprises at least 8 carbon atoms, for example from 10 to 30 carbon atoms, as a further example from 12 to 30 carbon atoms, and as yet a further example from 18 to 30 carbon atoms.

In certain embodiments, the thickening copolymer may be chosen from the copolymers resulting from the polymerization of:
(1) at least one monomer of formula (II):

   CH2=CH(R1)COOH (II)

   wherein R1 is chosen from H, CH3, or C2H5, providing acrylic acid, methacrylic acid, or ethacrylic acid monomers, and
(2) at least one monomer of (C10 C30)alkyl ester of unsaturated carboxylic acid type corresponding to the monomer of formula (III): CH2-CH(R2)COOR3(III)
wherein R2 is chosen from H, CH3, or C2H5, providing acrylate, methacrylate or ethacrylate units, and R3 denotes a C10 C30 alkyl radical, such as a C12 C22 alkyl radical.

In certain embodiments, the (C10 C30)alkyl esters of unsaturated carboxylic acids may be chosen from lauryl acrylate, stearyl acrylate, decyl acrylate, isodecyl acrylate, dodecyl acrylate or the corresponding methacrylates, such as lauryl methacrylate, stearyl methacrylate, decyl methacrylate, isodecyl methacrylate or dodecyl methacrylate, or mixtures thereof.

In certain embodiments, the crosslinked thickening polymer may be chosen from polymers resulting from the polymerization of a mixture of monomers comprising:
(1) acrylic acid,
(2) an ester of formula (III) described above, in which R2 is chosen from H or CH3, R3 denotes an alkyl radical having from 12 to 22 carbon atoms, and
(3) a crosslinking agent, which is a known copolymerizable polyethylenic unsaturated monomer, such as diallyl phthalate, allyl (meth)acrylate, divinylbenzene, (poly)ethylene glycol dimethacrylate and methylenebisacrylamide.

In various embodiments, the crosslinked thickening polymer may comprise from 60 % to 95 % by weight of acrylic acid (hydrophilic unit), from 4 % to 40 % by weight of C10 C30 alkyl acrylate (hydrophobic unit), and from 0 % to 6 % by weight of crosslinking polymerizable monomer. In further embodiments, the crosslinked thickening polymer may comprise from 96 % to 98 % by weight of acrylic acid (hydrophilic unit), from 1 % to 4 % by weight of C10 C30 alkyl acrylate (hydrophobic unit), and from 0.1 % to 0.6 % by weight of crosslinking polymerizable monomer, such as those described above.

In an embodiment, the anionic thickening agent is chosen from polyacrylate-3, commercially known under the trade name of Viscophobe DB-100 and sold by The Dow Chemical Company, carbomers, commercially known under the trade name of Carbopol polymers and sold by Lubrizol Advance Materials, Inc, acrylates/C10-30 alkyl acrylate crosspolymers, such as the products sold under the tradenames Pemulen^{™} TR1, Pemulen^{™} TR2, Carbopol^{®} 1382, Carbopol^{®} EDT 2020, and Carbopol^{®} Ultrez 20 Polymer by the company Lubrizol, Acrylates/C10-30 Alkyl Acrylate Crosspolymer such as by and sold by Lubrizol Advance Materials, Inc,, AMP-acrylates/allyl methacrylate copolymer, commercially known under the trade name of Fixate G-100 polymer and sold by Lubrizol Advance Materials, Inc., Polyacrylate Crosspolymer-6 such as SepimaxTM Zen by the company Seppic, and a crosslinked methacrylic acid/ethyl acrylate copolymer, also known as an acrylates copolymer in aqueous dispersion, such as the slightly cross-linked, alkali-swellable acrylate polymer known by the INCI name acrylates copolymer and sold by Lubrizol, under the tradename CARBOPOL Aqua SF-1 as an aqueous dispersion comprising 30 percent by weight of total solids or active material.

In certain embodiments, the thickening agents may be chosen from hydrophilic thickeners, for example cellulose polymers and gums, modified or unmodified carboxyvinyl polymers, such as those sold under the tradename Carbopol^{®} (CTFA name: carbomer) by the company Goodrich, polyacrylamides, copolymers of acrylic acid and of acrylamide sold in the form of the sodium salt thereof, such as those sold under the tradenames Reten^{®} by Hercules, and the sodium salts of polyhydroxycarboxylic acids such as those sold under the tradename Hydagen F^{®} by the company Henkel, polyacrylic acid/alkyl acrylate copolymers of Pemulen^{™} type, associative polymers, for instance PEG-150/stearyl alcohol/SMDI copolymer such those as sold under the tradename ACULYN^{™} 46 by the company Rohm & Haas, steareth-100/PEG-136/HDI copolymer such as those sold under the tradename Rheolate^{®} FX 1100 by the company Elementis, and mixtures thereof.

In certain embodiments, the hydrophilic thickener may be chosen from associative polymers. As used herein, the term "associative polymer" is intended to mean any amphiphilic polymer comprising in its structure at least one fatty chain and at least one hydrophilic portion. As used herein, the term "amphiphilic polymer" means a polymer composed of hydrophilic and hydrophobic parts.

In certain embodiments, the associative polymers may be anionic, cationic, nonionic, or amphoteric. In certain embodiments, the associative polymers may be chosen from polymers comprising at least one hydrophilic unit and at least one fatty-chain allyl ether unit; polymers in which the hydrophilic unit is constituted of an ethylenic unsaturated anionic monomer, such as a vinylcarboxylic acid, acrylic acid, methacrylic acid, or mixtures thereof; and polymers in which the fatty-chain allyl ether unit corresponds to the monomer of formula (I) below:

CH₂ = C(R')CH₂OBₙR (I)

in which R' is chosen from H or CH₃, B is an ethyleneoxy radical, n is zero or is chosen from an integer ranging from 1 to 100, and R is a hydrocarbon-based radical chosen from alkyl, arylalkyl, aryl, alkylaryl, or cycloalkyl radicals containing from 8 to 30 carbon atoms, from 10 to 24 carbon atoms, or from 12 to 18 carbon atoms. Exemplary and non-limiting polymers of this type are described and prepared, according to an emulsion polymerization process, in patent EP 0 216 479.

In other embodiments, the associative cationic polymer may be chosen from quaternized cellulose derivatives and polyacrylates containing amine side groups.

In other embodiments, the non-ionic associative polymer may be chosen from celluloses modified with groups comprising at least one fatty chain, for instance hydroxyethyl celluloses modified with groups comprising at least one fatty chain, such as alkyl groups, e.g. C₈-C₂₂ alkyl groups, arylalkyl and alkylaryl groups; cetyl hydroxyethyl cellulose, also known under the tradename Natrosol^{®} Plus (sold by the company Ashland), Bermocoll EHM 100 (sold by the company Berol Nobel), Amercell Polymer HM-1500^{®} sold by the company Amerchol; hydroxyethylcellulose modified with a polyethylene glycol (15) nonylphenyl ether group, sold by the company Amerchol; celluloses modified with polyalkylene glycol alkylphenyl ether groups; guars such as hydroxypropyl guar, optionally modified with groups comprising at least one fatty chain such as an alkyl chain, for example Jaguar^{®} XC-95/3 (C14 alkyl chain, sold by the company Rhodia Chimie), Esaflor HM 22 (C22 alkyl chain, sold by the company Lamberti), RE210-18 (C14 alkyl chain) and RE205-1 (C20 alkyl chain, sold by the company Rhodia Chimie); copolymers of vinylpyrrolidone and of fatty-chain hydrophobic monomers, for instance Antaron^{®} or Ganex^{®} V216 (vinylpyrrolidone/hexadecene copolymers), Antaron^{®} or Ganex^{®} V220 (vinylpyrrolidone/eicosene copolymers) sold by the company I.S.P.; copolymers of C₁-C₆ alkyl methacrylates, acrylates or amphiphilic monomers comprising at least one fatty chain; or copolymers of hydrophilic methacrylates, acrylates or hydrophobic monomers comprising at least one fatty chain, for instance the polyethylene glycol methacrylate/lauryl methacrylate copolymer.

Associative polyurethanes may also be chosen in various embodiments. As used herein, "associative polyurethanes" are nonionic block copolymers comprising in the chain both hydrophilic blocks usually of polyoxyethylene nature, and hydrophobic blocks that may be aliphatic sequences alone and/or cycloaliphatic and/or aromatic sequences. Associative polyurethanes comprise at least two hydrocarbon-based lipophilic chains containing from C₆ to C₃₀ carbon atoms, separated by a hydrophilic block, the hydrocarbon-based chains optionally being pendent chains or chains at the end of a hydrophilic block. For example, it is possible for one or more pendent chains to be provided. In addition, the polymer may comprise a hydrocarbon-based chain at one or both ends of a hydrophilic block. The associative polyurethanes may be arranged in triblock or multiblock form. The hydrophobic blocks may thus be at each end of the chain (for example, triblock copolymer with a hydrophilic central block) or distributed both at the ends and within the chain (for example, multiblock copolymer). These polymers may also be graft polymers or starburst polymers. In one embodiment, the associative polyurethanes may be triblock copolymers in which the hydrophilic block is a polyoxyethylene chain containing from 50 to 1000 oxyethylene groups.

In other embodiments, associative polymers of the polyurethane polyether type that may be used include the polymer C₁₆-OE₁₂₀-C₁₆ from Servo Delden (under the tradename SER AD FX1100), which is a molecule containing a urethane function and having a weight-average molecular weight of 1300, OE being an oxyethylene unit; Nuvis^{®} FX 1100 (European and US INCI name "Steareth-100/PEG-136/HMDI Copolymer" sold by the company Elementis Specialties); Acrysol RM 1840 (sold by the company Rohm and Haas); Elfacos^{®} T210^{®} (C12-C14 alkyl chain); Elfacos^{®} T212^{®} (C18 alkyl chain) sold by the company Akzo; Rheolate^{®} 205 containing a urea function, sold by the company Rheox; RHEOLATE^{®} 208 or 204, or RHEOLATE^{®} FX1100 sold by the company Elementis; or DW 1206B sold by the company Rohm & Haas containing a C₂₀ alkyl chain with a urethane bond, sold at a solids content of 20% in water.

In further embodiments, solutions or dispersions of these polymers, especially in water or in aqueous-alcoholic medium, may be chosen. Examples of such polymers include SER AD FX1010, SER AD FX1035, and SER AD 1070 from the company Servo Delden, and Rheolate^{®} 255, Rheolate^{®} 278, and Rheolate^{®} 244 sold by Rheox. Further examples include the products Aculyn^{™} 46, DW 1206F, and DW 1206J, Acrysol RM 184 or Acrysol 44 from the company Rohm & Haas, and Borchi^{®} Gel LW 44 from the company Borchers.

In further embodiments, the additonal thickening agent may be chosen from nonionic homopolymers or copolymers containing ethylenically unsaturated monomers of the amide type, for example, the polyacrylamide products sold under the tradenames Cyanamer^{®} P250 by the company CYTEC.

In further embodiments, the additional thickening agent chosen from polymers of natural origin may include thickening polymers comprising at least one sugar unit, for instance nonionic guar gums, optionally modified with C1-C6 hydroxyalkyl groups; biopolysaccharide gums of microbial origin, such as scleroglucan gum (also known as sclerotium gum) or xanthan gum; gums derived from plant exudates, such as gum arabic, ghatti gum, karaya gum, gum tragacanth, carrageenan gum, agar gum, carob gum, ceratonia siliqua gum or cyamopsis tetragonoloba (guar) gum; pectins; alginates; starches; hydroxy(C1-C6)alkylcelluloses; or carboxy(C1-C6)alkylcelluloses.

In certain embodiments, the nonionic, unmodified guar gums may be chosen from Guargel D/15 sold by the company Noveon, Vidogum GH 175 sold by the company Unipectine, Meypro-Guar 50 sold by the company Meyhall, or Jaguar@ C sold by the company Rhodia Chimie. In other embodiments, the nonionic modified guar gums may be chosen from Jaguar@ HP8, HP60, HP120, DC 293 and HP 105 sold by the companies Meyhall and Rhodia Chimie or Galactasol^{™} 4H4FD2 sold by the company Ashland.

In other embodiments, the additional thickening agents may be chosen from scleroglucans, for example, Actigum^{™} CS from Sanofi Bio Industries; Amigel^{®} sold by the company Alban Muller International; xanthan gums, for instance Keltrol^{®}, Keltrol^{®} T, Keltrol^{®} Tf, Keltrol^{®} Bt, Keltrol^{®} Rd, and Keltrol^{®} Cg sold by the company CP Kelco, Rhodicare^{®} S and Rhodicare^{®} H sold by the company Rhodia Chimie; starch derivatives, for instance Primogel^{®} sold by the company Avebe; hydroxyethylcelluloses such as Cellosize^{®} QP3L, QP4400H, QP30000H, HEC30000A and Polymer PCG10 sold by the company Amerchol, Natrosol^{™} 250HHR, 250MR, 250M, 250HHXR, 250HHX, 250HR, and 250 HX, sold by the company Hercules, or Tylose^{®} H1000 sold by the company Hoechst; hydroxypropylcelluloses, for instance Klucel^{™} EF, H, LHF, MF, and G, sold by the company Ashland; carboxymethylcelluloses, for instance Blanose^{®} 7M8/SF, refined 7M, 7LF, 7MF, 9M31F, 12M31XP, 12M31P, 9M31XF, 7H, 7M31, and 7H3SXF, sold by the company Ashland, Aquasorb^{®} A500 sold by the company Hercules, Ambergum^{®} 1221 sold by the company Hercules, Cellogen^{®} HP810A and HP6HS9 sold by the company Montello and Primellose^{®} sold by the company Avebe.

In other embodiments, the modified nonionic guar gums may, for example, be modified with C1-C6 hydroxyalkyl groups. Such hydroxyalkyl groups may be chosen from hydroxymethyl, hydroxyethyl, hydroxypropyl, and hydroxybutyl groups.

In certain embodiments, guar gums may be prepared by reacting the corresponding alkylene oxides, such as for example propylene oxides, with guar gum so as to obtain a guar gum modified with hydroxypropyl groups. The hydroxyalkylation ratio, which corresponds to the number of alkylene oxide molecules consumed to the number of free hydroxyl functional groups present on the guar gum, may in certain embodiments range from 0.4 to 1.2.

Examples of nonionic guar gums, optionally modified with hydroxyalkyl groups, include those sold under the tradenames Jaguar@ HP8, Jaguar@ HP60, Jaguar^{®} HP120, Jaguar@ DC 293, and Jaguar^{®} HP 105 by the company Rhodia Chimie, and under the tradename Galactasol^{™} 4H4FD2 by the company Ashland.

In other embodiments, the guar gum may be chosen from those modified with a quaternary ammonium group, such as guar hydroxypropyltrimonium chloride, also sold under the tradename Jaguar^{®} C-13S by the company Rhodia Chimie.

In other embodiments, the ceulloses may be chosen from hydroxyethylcelluloses and hydroxypropylcelluloses, such as those sold under the tradenames Klucel^{™} EF, Klucel^{™} H, Klucel^{™} LHF, Klucel^{™} MF, Klucel^{™} G, by the company Ashland and under the tradename Cellosize^{™} PCG-10 by the company Amerchol.

In other embodiments, non-limiting thickening polysaccharides may be chosen from glucans; modified or unmodified starches, for example, Hydroxypropyl Starch Phosphate, sold under the tradename of STURCTURE ZEA by Akzo Nobel or such as those derived, for example, from cereals such as wheat, corn or rice, vegetables such as golden pea, or tubers such as potato or cassava; amylose, amylopectin, glycogen, dextrans, celluloses or derivatives thereof (methylcelluloses, hydroxyalkylcelluloses, ethylhydroxyethylcelluloses), mannans, xylans, lignins, arabans, galactans, galacturonans, chitin, chitosans, glucoronoxylans, arabinoxylans, xyloglucans, glucomannans, pectic acids or pectins, arabinogalactans, carrageenans, agars, gums arabic, gums tragacanth, Ghatti gums, Karaya gums, carob gums, galactomannans such as guar gums and their nonionic derivatives such as hydroxypropylguar, or mixtures thereof.

In certain embodiments, the additional thickening agent may be chosen from silicas or hydrophobic silicas, such as those described in EP-A-898960. Examples of such silicas include those sold under the tradename Aerosil^{®} R812 by the company Degussa, CAB-O-SIL^{®} TS-530, CAB-O-SIL^{®} TS-610, CAB-O-SIL^{®} TS-720 by the company Cabot, or Aerosil^{®} R972 and Aerosil^{®} R974 by the company Degussa; clays, such as montmorillonite; modified clays such as the bentones, for example, stearalkonium hectorite, stearalkonium bentonite; or polysaccharide alkyl ethers, optionally with the alkyl group having from 1 to 24 carbon atoms, for example from 1 to 10 carbon atoms, from 1 to 6 carbon atoms, or from 1 to 3 carbon atoms, such as those described in document EP-A-898958.

In certain embodiments, when an anionic thickening agent is used, it is generally neutralized before being included in, or as it is added to the compositions of the disclosure. Such an anionic thickening agent may be neutralized by employing traditional neutralizing agents such as alkanolamines, for example, monoethanolamine and diethanolamine; aminomethyl propanol; basic amino acids, for example arginine and lysine; or ammonium compounds and their salts. The anionic thickening agent may also be neutralized by at least one latex polyurethane polymer of the disclosure wherein said latex polyurethane polymer has at least one free amino group and/or is provided in a dispersion medium that has a pH of greater than 7.

In certain embodiments, the additional thickening agent may be chosen from non-associative cationic polymers;

In one embodiment, the additional thickening agent is chosen from xanthan gum, guar gum, hydroxypropyl guar, guar hydroxypropyl trimonium chloride, hydroxyethyl cellulose, hydroxypropyl cellulose, cetyl hydroxyethyl cellulose, hydroxypropyl starch phosphate, ammonium acryloyldimethyltaurate/VP copolymer or mixtures thereof.

In various embodiments, the additional thickening agent may be present in an amount ranging from 0.01% to 10% by weight, or from 0.05% to 5% by weight, or from 0.1% to 3% by weight, relative to the total weight of the composition, including all ranges and subranges therebetween.

ln various embodiments, the additional thickening agent may be present in an amount of 0.01%, 0.02%, 0.03%, 0.04%, 0.05%, 0.06%, 0.07%, 0.075%, 0.08%, 0.09%, 0.1%, 0.125%, 0.15%, 0.2%, 0.25% 0.3%, 0.325%, 0.35%, 0.375%, 0.4%, 0.425%, 0.45%, 0.5%,0.55%, 0.6%, 0.65%, 0.7%, 0.75%, 0.8%, 0.85%, 0.9%, 0.95%, 1%, 1.5%, 2%, 2.5%, or 3% by weight, relative to the total weight of the composition.

### COALESCING AGENTS AND PLASTICIZERS

The compositions according to the disclosed embodiments may optionally comprise at least one component chosen from coalescing agents and plasticizers. Without wishing to be bound by theory, it is believed that the addition of coalescing agents and/or plasticizers may lower the glass transition temperature (Tg), decrease the Young's modulus, and/or increase the strain of latex polymers and/or the films formed by latex polymers. Further, the at least one coalescing agent and/or plasticizer may also be used to aid coating formation of the latex film to form a continuous and homogeneous film or coating and to improve adhesion. While the lowering of the Tg of the latex polymers can result in a softening of the film or coating formed by the latex polymers, it has been found that the coating or film produced on hair treated with the compositions of the disclosure imparts a stronger styling hold to the hair and a more balanced coating or film.

In various embodiments, the coalescing agents and/or plasticizers may be chosen from glycols and their derivatives, such as glycol ethers, for example, ethylene glycol, propylene glycol, diethylene glycol ethyl ether, diethylene glycol methyl ether, diethylene glycol butyl ether, diethylene glycol hexyl ether, diethylene glycol dibutyl ether, ethylene glycol methyl ether, ethylene glycol ethyl ether, ethylene glycol butyl ether, or ethylene glycol hexyl ether; glycol esters, such as diethylene glycol butyl ether acetate, propylene glycol dibenzoate or dipropylene glycol dibenzoate; cellulose esters, such as sucrose acetate; propylene glycol derivatives, such as propylene glycol phenyl ether, propylene glycol diacetate, dipropylene glycol butyl ether, tripropylene glycol butyl ether, propylene glycol methyl ether, dipropylene glycol ethyl ether, tripropylene glycol methyl ether, diethylene glycol methyl ether, or propylene glycol butyl ether.

In other embodiments, the coalescing agents and/or plasticizers may be chosen from acid esters, such as carboxylic acid esters. In other embodiments, the component chosen from coalescing agents and plasticizers may be chosen from acetates, such as glycerol triacetate; citrates, such as triethyl citrate, tributyl citrate, triethyl acetylcitrate, tributyl acetylcitrate, or tri(2-ethylhexyl)acetylcitrate; phthalates, such as diethyl phthalate, dibutyl phthalate, dioctyl phthalate, dipentyl phthalate, dimethoxyethyl phthalate, butyl phthalate, or 2-ethylhexyl phthalate; phosphates, such as tricresyl phosphate, tributyl phosphate, triphenyl phosphate, or tributoxyethyl phosphate; tartrates, such as dibutyl tartrate; or sebacates, such as dimethyl sebacate or dibutyl sebacate.

In other embodiments, the coalescing agents and/or plasticizers may be chosen from, fatty acid esters, such as adipic acid esters, for example, diisobutyl adipate or diethyl adipate; stearic acid esters, such as ethyl stearate; or palmitic acid esters, such as 2-ethylhexyl palmitate, succinates, abietates, caprylates, caproates, enanthates, or myristates.

In further embodiments, the coalescing agents and/or plasticizers may be chosen from carbonates, such as ethylene carbonate or propylene carbonate; benzyl benzoate, sucrose benzoate, butyl acetylricinoleate, glyceryl acetylricinoleate, butyl glycolate, camphor, N-ethyl-o,p-toluenesulphonamide, or ethyl tosylamide.

In further embodiments, the coalescing agents and/or plasticizers may be chosen from oxyethylenated derivatives, such as oxyethylenated oils, for example, vegetable oil, castor oil, oils of natural origin, including non-drying oils, or those comprising at least one fatty acid chosen from caprylic acid, capric acid, lauric acid, myristic acid, palmitic acid, stearic acid, oleic acid, ricinoleic acid, linoleic acid, linolenic acid, arachidic acid, gadoleic acid, behenic acid, erucic acid, brassidic acid, cetoleic acid, lignoceric acid, or nervonic acid.

In certain embodiments, the oils may be chosen from triglycerides composed of esters of fatty acids and of glycerol, of which the fatty acids have varied chain lengths from C4 to C24 that can be linear or branched and saturated or unsaturated.

in other embodiments, the oils may be chosen from heptanoic or octanoic triglycerides, triglycerides of caprylic/capric acids, or oils derived from groundnut, babassu, coconut, grape seed, cottonseed, maize, maize germ, mustard seed, palm, rapeseed, sesame, soybean, sunflower, wheat germ, canola, apricot, mango, castor, shea, avocado, olive, sweet almond, almond, peach, walnut, hazelnut, macadamia, jojoba, alfalfa, poppy, pumpkinseed, cucumber, blackcurrant, evening primrose, millet, barley, guinea, rye, safflower, candlenut, passionflower, musk rose, or shea butter. In further embodiments, the oils may be chosen from alcohols such as hexanol and benzyl alcohol.

In preferred embodiments, the coalescing agents and/or plasticizers may be chosen from propylene glycol dibenzoate, sold under the tradename Lexfeel^{®} Shine by the company Inolex, dipropylene glycol dibenzoate, sold under the tradename Dermol DPG-2b by the company Alzo, and propylene glycol butyl ether, sold under the tradename Dowanol^{™} PnB by the company Dow Chemical.

It should be understood that mixtures of the above agents may be used according to various embodiments.

In various embodiments, the at least one component chosen from coalescing agents and plasticizers may be present in an amount ranging from 0.1% to 20% by weight, from 0.1% to 10% by weight, or from 0.1% to 5% by weight, with respect to the total weight of the composition.

In various embodiments, the at least one component chosen from coalescing agents and plasticizers may be present in an amount ranging from 0.1% to 2% by weight or from 0.1% to 1% by weight, with respect to the total weight of the composition.

In other embodiments, compositions of the disclosure may comprise at least one water-soluble resin such as polyethylene oxide having a molecular weight ranging from 100,000 to 10,000,000. Examples of such polyethylene oxides include, but are not limited to, Polyox water-soluble resins manufactured by the company Dow under the INCI names of PEG-2M, PEG-5M, PEG-7M, PEG-14M, PEG-23M, PEG-45M, PEG-90M, PEG-160M, and PEG-180M. PEG-90M is known under the tradename Polyox^{™} WSR 301 and PEG-45M is known under the tradename Polyox^{™} WSR 60k. The amounts of water-soluble resins in the compositions, when present, may range from 0.1% to 2% by weight, relative to the total weight of the composition.

It is to be understood that any combination of the above mentioned agents is contemplated according to various exemplary embodiments of the disclosure.

According to various embodiments, the at least one thickening agent may be present in an amount ranging from 0.01% to 10% by weight, from 0.1% to 5% by weight, from 0.2% to 4% by weight, or from 0.5% to 2% by weight, relative to the total weight of the composition.

### FATTY ESTER

The compositions of the present invention may further comprise at least one fatty ester other than plant oils, preferably containing from 12 to 50 carbon atoms.

In one embodiment, the fatty esters are solid fatty esters of monoalcohols, especially of monoalcohols comprising at least 10 carbon atoms, and better still of saturated monoalcohols comprising at least 10 carbon atoms or from 10 to 30 carbon atoms and more particularly from 12 to 24 carbon atoms. Solid fatty esters are esters of saturated carboxylic acids comprising at least 10 carbon atoms and of saturated monoalcohols comprising at least 10 carbon atoms or from 10 to 30 carbon atoms and more particularly from 12 to 24 carbon atoms. The saturated carboxylic acids and/or monoalcohols may be linear or branched or optionally be hydroxylated.

Solid fatty esters may be chosen from myristyl myristate, cetyl myristate, stearyl myristate, myristyl palmitate, cetyl palmitate, stearyl palmitate, myristyl stearate, cetyl stearate and stearyl stearate, and also mixtures thereof.

The fatty ester(s) are preferably present in the composition in an amount of between 0.1% and 10% by weight and preferably between 0.5% and 5% by weight relative to the total weight of the composition.

### SOLVENT

In addition to a solvent chosen from water, the compositions according to the disclosed embodiments may further comprise at least one cosmetically acceptable organic solvent. In certain embodiments, the at least one solvent in the compositions of the invention may be chosen from water, at least one cosmetically acceptable organic solvent, or a mixture of water and at least one cosmetically acceptable organic solvent. The cosmetically acceptable organic solvent may be chosen from volatile and non-volatile organic solvents.

In various embodiments, the cosmetically acceptable organic solvents may be water-miscible, e.g. a mixture capable of forming at 25°C a homogeneous mixture that is transparent, or substantially transparent, to the eye, chosen from lower monoalcohols, such as those containing from 1 to 5 carbon atoms, for example ethanol and isopropanol; polyols, including glycols, such as those containing from 2 to 8 carbon atoms, for example propylene glycol, ethylene glycol, 1,3-butylene glycol, dipropylene glycol, pentylene glycol, hexylene glycol, glycerin, ethylhexylglycerin; hydrocarbons, such as, for example, isododecane and mineral oil; silicones, such as dimethicones, trisiloxanes, cyclomethicones, and cyclopentasiloxane; or mixtures thereof.

In some embodiments, the cosmetically acceptable organic solvent is chosen from propylene glycol, glycerin, ethylhexylglycerin, trisiloxane, dimethicone, isododecane, mineral oil, and mixtures thereof.

In certain embodiments, the latex polymer particles are not soluble in the solvent of the composition, and thus remain in particulate form while in the composition and after evaporation of the solvent. For example, in embodiments where the composition comprises alcohol as a cosmetically acceptable organic solvent, the latex particles may remain in particulate form upon evaporation of the alcohol, such as once the composition is applied to a substrate.

According to various embodiments, the at least one solvent may be present in an amount ranging up to 95% by weight, from 1% to 90% by weight, or from 5% to 80% by weight, relative to the total weight of the composition.

### PROPELLANT

The compositions according to the disclosed embodiments may further comprise at least one propellant. As used herein, the term "propellant" is meant to indicate a liquid or gas that is packaged with the composition in a device under pressure, which serves to dispense the composition from the device with force and/or facilitate or enhance the foaming of the composition.

Nonlimiting examples of propellants that are suitable for use include gases usually used in the cosmetic field, in particular optionally halogenated volatile hydrocarbons, for example n-butane, propane, isobutane, or pentane, and halogenated derivatives thereof; carbon dioxide, nitrous oxide, dimethyl ether, hydrofluorocarbons, and nitrogen, alone or as mixtures.

In certain embodiments of the disclosure, the propellant is chosen from alkanes and in particular from n-butane, propane, and isobutane, and mixtures thereof.

According to various embodiments, the propellant is under pressure, and at least partially in liquid form.

In certain embodiments, the total amount of propellant ranges from 1 % to 30 % by weight, relative to the weight of the composition, such as from 2 % to 15 % by weight relative to the weight of the composition.

### COMPOSITIONS

The compositions according to the disclosed embodiments may further comprise additional components that are typically used in hair styling compositions. Such components are known to those of skill in the art, or are within the ability of those of skill in the art to determine depending on the particular application, such as, for example, organic amines, carbonate compounds, surfactants (amphoteric, cationic, anionic) fillers, pigments, conditioning agents, moisturizing agents, shine agents, sequestering agents, fragrances, preservatives, pH modifiers/neutralizing agents, stabilizers, propellants, or mixtures thereof.

In various embodiments, the composition described herein may have a pH ranging from 2 to 9, such as 3 to 8, or 5 to 7.

In some embodiments, the compositions are in the form of hair styling compositions, in any form, such as, for example, a gel, a cream, a foam, a lotion, an emulsion, or a liquid that may be sprayed onto or otherwise applied to the hair. In certain exemplary embodiments, the composition is provided in the form of a cream or thick lotion.

In various embodiments, the composition is a hair styling composition. According to various embodiments, by "hair styling composition" the composition is meant to be applied to hair on the head other than eyelashes and/or eyebrows. Hair styling compositions and mascaras are sometimes distinguishable based on the components of the compositions and/or the effects of the compositions when applied. In some embodiments, at least one component of a hair styling composition is not compatible for use in a mascara. In other embodiments, at least one component of a mascara is not compatible for use in a hair styling composition.

According to some embodiments, the composition is not applied to the eyelashes and/or eyebrows. In certain embodiments, the composition is not a mascara.

In certain embodiments, the composition may be applied to the hair by first applying to the hands, and then contacting the hair with the hands. In other embodiments, the composition may be applied directly onto the hair, such as by spraying. In other embodiments, the composition may be applied to wet or dry hair. The compositions may, in various embodiments, be applied to the hair as a leave-on treatment.

Also disclosed herein are methods for styling or shaping the hair, said methods comprising applying a composition according to the disclosure to the hair, either before, during, or after styling or shaping the hair.

In one embodiment, the hair is allowed to air dry after application of the composition, and no heat is applied to the hair.

In one embodiment, the hair is allowed to air dry after application of the composition, and is styled or shaped with no heat being applied to the hair.

Styling or shaping the hair may involve the use of devices on hair such as a brush, a comb or running the fingers of the hand through the hair.

In one embodiment, the application of an external stimuli, such as heat, may be part of the hair styling process. By way of example only, before, during, or after the composition is applied to wet or dry hair, the hair may optionally be further treated with an external stimuli, for example with heat ranging from 25°C to 250°C. In at least certain embodiments, the hair may also be shaped or positioned as desired while exposed to external stimuli, such as while heated or exposed to heat.

Professional and consumer heating tools can be used as a means to deliver heat or an elevated temperature to the hair. The heating tools can generate heat through electrical current or heating lamps. Depending upon the desired style, these tools include, but are not limited to, heaters, blow dryers, flat irons, hot combs, hot curler sets, heated crimpers, heated lash curlers, heated wandslbrushes, and hood driers or their combinations thereof.

As described, compositions according to the disclosure may impart a film on a substrate, such as on the hair or on the hand during or after application to the hair. A film formed by the composition according to certain embodiments may be surprisingly clean-feeling and not sticky, as with traditional hair styling compositions. Also surprisingly, the composition may impart a film on the hair that leaves the hair relatively natural and clean-feeling, yet has a flexible coating, leaving little to no residue, allows the hair to be bouncy and springy with little to no frizz or flaking, may impart relatively high definition with individualized curls, style control, volume, and/or shine, and/or may allow for relatively long-lasting hold and style memory. Furthermore, in at least certain embodiments according to the disclosure, the compositions are not sticky or tacky. A user of hair compositions according to various embodiments described herein may thus feel that the composition is not perceptible or is "invisible," yet still effectively style and/or hold the hair. Additionally, the compositions may have effective hair styling and/or hold properties, even in conditions of high, or relatively high, humidity. In at least certain embodiments according to the disclosure, the compositions may be quick-drying, which may allow drying and/or styling time to be reduced, as well as further improve ease of styling and curl retention.

Furthermore, as described, compositions prepared according to the disclosed embodiments may provide for varying degrees of hold to be imparted to a hair style. By way of non-limiting example only, in order to obtain a spiky look to hair of a very short length, a high level of styling hold may be desirable. Or, as a further non-limiting example, in order to obtain a flowing look or to maintain hair curls for hair of medium length or longer length, a light to medium level of style hold may be desirable. By altering the weight ratio of the first and second polymers, it is possible to formulate compositions having high levels of style hold, medium to high levels of style hold, medium levels of style hold, light to medium levels of style hold, or light level of style hold.

It was also surprisingly discovered that by varying the levels and/or nature of the latex polymers or the thickening agents or the aminofunctional silicones or by adding additional non-latex nonionic film forming polymers, various styling and shaping effects and cosmetic attributes can be obtained on hair.

In at least certain embodiments, a film formed by the compositions described herein may be clear and/or stable. In such embodiments, phase separation and dramatic aggregation are minimized.

In addition, hair styled or treated with compositions according to the disclosure, in at least certain exemplary embodiments, may be hydrophobic, may appear less frizzy, and/or may be less prone to breakage, relative to hair subjected to the same conditions but not having been styled or treated with a composition according to the disclosure.

It should be noted, however, that compositions and films, as well as hair to which the composition or film has been applied, according to the disclosure may not have one or more of the herein-referenced properties, yet are intended to be within the scope of the disclosure.

It is to be understood that both the foregoing description and the following Examples are exemplary and explanatory only, and are not to be interpreted as restrictive of the disclosure. Moreover, it should be understood that various features and/or characteristics of differing embodiments herein may be combined with one another. Other embodiments will be apparent to those skilled in the art from consideration of the disclosure and practice of the various exemplary embodiments disclosed herein.

It is also to be understood that, as used herein the terms "the," "a," or "an," mean "at least one," and should not be limited to "only one" unless explicitly indicated to the contrary. Thus, for example, the use of "a surfactant" is intended to mean at least one surfactant unless the context clearly indicates otherwise.

While various features, elements or steps of particular embodiments may be disclosed using the transitional phrase "comprising," it is to be understood that alternative embodiments, including those that may be described using the transitional phrases "consisting" or "consisting essentially of," are implied. Thus, for example, implied alternative embodiments to a method that comprises A+B+C include embodiments where a method consists of A+B+C and embodiments where a method consists essentially of A+B+C. As described, the phrase "at least one of A, B, and C" is intended to include "at least one A or at least one B or at least one C," and is also intended to include "at least one A and at least one B and at least one C."

All ranges and amounts given herein are intended to include subranges and amounts using any disclosed point as an end point. Thus, a range of "1% to 10%, such as 2% to 8%, such as 3% to 5%," is intended to encompass ranges of "1% to 8%," "1% to 5%," "2% to 10%".

Unless otherwise expressly stated, it is in no way intended that any method set forth herein be construed as requiring that its steps be performed in a specific order. Accordingly, where a method claim does not expressly recite an order to be followed by its steps or it is not otherwise specifically stated in the claims or descriptions that the steps are to be limited to a specific order, it is no way intended that any particular order be inferred.

It should be understood that compositions according to various embodiments of the disclosure form a film when applied to a substrate. However, the various properties of the film described herein are intended to include any film provided by compositions according to the disclosure, regardless of whether the film is attached or bonded to the substrate or not. By way of example only, once the compositions are applied to a substrate and a film is formed, the film may subsequently be removed in order to evaluate properties such as strain and Young's modulus.

Notwithstanding that the numerical ranges and parameters setting forth the broad scope of the disclosure are approximations, unless otherwise indicated the numerical values set forth in the specific examples are reported as precisely as possible. Any numerical value, however, inherently contains certain errors necessarily resulting from the standard deviation found in their respective testing measurements. The example that follows serves to illustrate embodiments of the present disclosure without, however, being limiting in nature.

It is understood that when an amount of a component is given, it is intended to signify the amount of the active material, unless otherwise indicated.

The compositions and methods according to the present disclosure can comprise, consist of, or consist essentially of the elements and limitations described herein, as well as any additional or optional ingredients, components, or limitations described herein or otherwise known in the art.

### EXAMPLES

The following Examples are intended to be non-restrictive and explanatory only, with the scope of the disclosed embodiments.

The ingredient amounts in the compositions/formulas described below are expressed in % by weight of active material ("AM") unless otherwise specificed, based on the total weight of the composition.

The formulation examples below contain 2 latex polymers, 1 or 2 aminofunctional silicones, thickening agent, and nonionic surfactants

**Table 1 Hair composition with 2 latex polymers, 2 aminofunctional silicones, thickening agent, nonionic surfactants, and organic solvents**

| US INCI NAME | Example 1 |
|---|---|
| Acrylates copolymer | 0.5 |
| Polyurethane-34 | 0.5 |
| PEG-40/PPG-8 Methylaminopropyl/hydroxylpropyl dimethicone copolymer | 0.6 |
| Trideceth-9 PG Amodimethicone | 0.35 |
| Thickening agent: Polyacrylamide (and) C13-14 Isoparaffin (and) Laureth-7 (Sepigel^{™} 305 from Seppic) | 1.5 |
| Glyceryl Stearate | 0.5 |
| PEG-75 Lanolin | 0.5 |
| Cetearyl Alcohol | 0.37 |
| PEG-40 Hydrogenated Castor Oil | 1.5 |
| Propylene Glycol | 2 |
| Glycerin | 0.68 |
| Trisiloxane | 1 |
| Dipropylene Glycol | 0.22 |
| Nonionic surfactants (Polysorbate 80, Trideceth-12) | 0.1 |
| Additional Components (e.g., anionic surfactants, cyclodextrin, t-butyl alcohol, preservatives, neutralizing agents, salt, and fragrances) and water | as 100 |

It can be contemplated that the aminofunctional silicones, PEG-40/PPG-8 Methylaminopropyl/hydroxylpropyl dimethicone copolymer and/or Trideceth-9 PG Amodimethicone , can be replaced by another aminofuncitonal silicone such as Amodimethicone and/or Amodimethiconel Morpholinomethylsilsesquioxane copolymer in Example 1.

The formulation example 1 was tested on hair on human heads. Performance of the formulas on hair are the following:
- Enhanced hair's natural shape and definition
- Performed as a conditioning styling cream
- Added softness
- Added shine
- Provided a styling hold for a controlled look without weighing the hair down
- Provided 24hr shape control
- No residue on hair
- No Flakes on hair
- No crunch feeling on hair
- Frizz resistant
- Touchable feel
Table 2 Hair composition with higher amounts 2 latex polymers, 2 aminofunctional silicones, higher amount of thickening agent, nonionic surfactants, and organic solvents

| US INCI NAME | Example 2 |
|---|---|
| Acrylates copolymer | 1 |
| Polyurethane-34 | 1 |
| PEG-40/PPG-8 Methylaminopropyl/hydroxylpropyl dimethicone copolymer | 0.6 |
| Trideceth-9 PG Amodimethicone | 0.35 |
| Thickening agent: Polyacrylamide (and) C13-14 Isoparaffin (and) Laureth-7 (Sepigel^{™} 305 from Seppic) | 2.7 |
| Glyceryl Stearate | 0.5 |
| PEG-75 Lanolin | 1 |
| Cetearyl Alcohol | 0.37 |
| PEG-40 Hydrogenated Castor Oil | 1.5 |
| Propylene Glycol | 2 |
| Glycerin | 0.68 |
| Dipropylene Glycol | 0.22 |
| Nonionic surfactants (Polysorbate 80, Trideceth-12, and Trideceth-10) | 0.12 |
| Additional Components (e.g., anionic surfactants, cyclodextrin, t-butyl alcohol, preservatives, neutralizing agents, salt, and fragrances) and Water | QS 100 |

It can be contemplated that the aminofunctional silicones, PEG-40/PPG-8 Methylaminopropyl/hydroxylpropyl dimethicone copolymer and/or Trideceth-9 PG Amodimethicone , can be replaced by another aminofuncitonal silicone such as Amodimethicone and/or Amodimethicone/Morpholinomethylsilsesquioxane copolymer in Example 2.

The formulation example 2 was tested on hair on human heads. Performance of the formulas on hair are the following:
- imparted good curl definition
- provided long lasting style/shape
- held down flyaways and frizz
- provided frizz control even in humid conditions

**Table 3 Hair Composition with 2 latex polymers, 1 aminofunctional silicone, thickening agent, nonionic surfactants, polymers (film forming polymers and thickening polymers), additional thickening agent, and organic solvent**

| US INCI NAME | Example 3 |
|---|---|
| Acrylates copolymer | 0. 5 |
| Polyurethane-34 | 0.5 |
| Trideceth-9 PG Amodimethicone | 0.35 |
| Thickening agent: Polyacrylamide (and) C13-14 Isoparaffin (and) Laureth-7 (Sepigel^{™} 305 from Seppic) | 0.5 |
| Glyceryl Stearate | 0.5 |
| Cetearyl Alcohol | 1.6 |
| PEG-40 Hydrogenated Castor Oil | 0.5 |
| Cetearyl Glucoside | 0.4 |
| Hydroxypropyl Starch Phosphate (additional thickening agent) | 1.32 |
| Propylene Glycol | 1 |
| Glycerin | 0.68 |
| Trisiloxane | 1 |
| Dipropylene Glycol | 0.22 |
| VP/VA copolymer (film forming polymer) | 0.5 |
| Ammonium AcryloyldimethyltaurateNP Copolymer (additional thickening agent) | 0.48 |
| Nonionic surfactants (Polysorbate 80, Trideceth-12, and Trideceth-1 0) | 0.1 |
| Additional Components (e.g., anionic surfactants, cyclodextrin, t-butyl alcohol, preservatives, neutralizing agents, salt, and fragrances) | 1.51 |
| Water qs | 100 |

It can be contemplated that the aminofunctional silicone, Trideceth-9 PG Amodimethicone , can be replaced by another aminofuncitonal silicone such as Amodimethicone and/or Amodimethiconel

Morpholinomethylsilsesquioxane copolymer or PEG-40/PPG-8 Methylaminopropyl/hydroxylpropyl dimethicone copolymer or mixtures thereof in Example 3.

The formulation examples were tested on hair on human heads Performance of the formulas on hair are the following:
- provided natural/clean feel
- frizz control
- provided long lasting shape control
- curl definition
- touchable feel

**Table 4 Hair Composition with 2 latex polymers, 1 aminofunctional silicone, thickening agent, nonionic surfactants, additional thickening agent, and organic solvent**

| US INCI NAME | Example 4 |
|---|---|
| Acrylates copolymer | 0. 5 |
| Polyurethane-34 | 0.5 |
| Trideceth-9 PG Amodimethicone | 2 |
| Thickening agent: Polyacrylamide (and) C13-14 Isoparaffin (and) Laureth-7 (Sepigel^{™} 305 from Seppic) | 0.5 |
| PEG-75 Lanolin | 0.2 |
| PEG-40 Hydrogenated Castor Oil | 1 |
| Propylene Glycol | 0.5 |
| Trisiloxane | 1 |
| Ammonium Acryloyldimethyltaurate/VP Copolymer (additional thickening agent) | 0.95 |
| Nonionic surfactants (Polysorbate 80, Trideceth-12, and Trideceth-10) | 0.35 |
| Additional Components (e.g., anionic surfactants, cyclodextrin, t-butyl alcohol, preservatives, neutralizing agents, salt, and fragrances) and water | QS 100 |

It can be contemplated that the aminofunctional silicone, Trideceth-9 PG Amodimethicone , can be replaced by another aminofuncitonal silicone such as Amodimethicone and/or Amodimethicone/

Morpholinomethylsilsesquioxane copolymer or PEG-40/PPG-8 Methylaminopropyl/hydroxylpropyl dimethicone copolymer or mixtures thereof in Example 4.

The formulation examples were tested hair on human heads. Performance of the formulas on hair are the following:
- hair feels light
- style looks effortless
- hair feels clean with no residue
- no flakes
- hair feels conditioned
- shape is defined
- touchable feel
- natural look and feel

The formulation examples above were prepared based on the following general protocol:
Water was added and heated in main tank. Then fatty alcohols (nonionic surfactants) were heated and melted in a separate beaker and added to the heated main tank. Once an emulsion was formed, the emulsion was cooled down. The latex polymers were then added. Preservatives, silicones, and fragrance were also added. The thickening agent was added last.

## Claims

1. A cosmetic composition comprising:
at least two latex polymers, wherein at least one latex polymer is a film-forming polymer;
at least one aminofunctional silicone chosen from amodimethicone/morpholinomethyl silsesquioxane copolymer, PEG-40/PPG-8 methylaminopropyl/hydroxypropyl dimethicone copolymer, trideceth-9 PG-amodimethicone, or mixtures thereof;
at least one thickening agent chosen from optionally crosslinked and/or neutralized 2-acrylamido-2-methylpropanesulfonic acid polymers (AMPS) and copolymers, crosslinked anionic copolymers of acrylamide, crosslinked anionic copolymers of AMPS, emulsions of crosslinked anionic copolymers of acrylamide/nonionic surfactants, emulsions of AMPS/nonionic surfactants, or mixtures thereof;
at least one nonionic surfactant chosen from glyceryl esters, fatty alcohols, alkoxylated alcohols and lanolin, alkylpolyglucosides, or mixtures thereof; and
water;
wherein the at least two latex polymers are present in a combined amount ranging from 0.1% to 30% by weight, particularly from 0.25% to 20% by weight, relative to the weight of the composition, and
wherein the at least one aminofunctional silicone is present in an amount ranging from 0.25% to 3% by weight, relative to the total weight of the composition.

2. The cosmetic composition according to claim 1, wherein the at least two latex polymers comprise at least one acrylate latex polymer and at least one polyurethane latex polymer, preferably wherein the at least two latex polymers comprise acrylates copolymer and polyurethane-34.

3. The cosmetic composition according to claim 2, wherein the weight ratio of the at least one acrylate latex polymer to the at least one polyurethane latex polymer ranges from 10:1 to 1:10, particularly from 5:1 to 1:5, most particularly 1.

4. The cosmetic composition according to any one of the preceding claims, wherein the at least one thickening agent is present in an amount ranging from 0.05% to 10% by weight, relative to the total weight of the composition, particularly from 0.3% to 5% by weight, relative to the total weight of the composition.

5. The cosmetic composition according to any one of the preceding claims, wherein the at least one thickening agent is chosen from a water-in-oil emulsion of Polyacrylamide/C13-14 Isoparaffin/Laureth-7, a water-in-oil emulsion of Acrylamide/Sodium acryloyldimethyltaurate copolymer/lsohexadecane/Polysorbate 80, or mixtures thereof.

6. The cosmetic composition according to any one of the preceding claims, wherein the at least one nonionic surfactant is present in an amount ranging from 0.05% to 8% by weight, relative to the total weight of the composition, particularly from 0.3% to 5% by weight, relative to the total weight of the composition.

7. The cosmetic composition according to any one of the preceding claims, wherein the at least one nonionic surfactant includes glyceryl esters chosen from glyceryl oleate, glyceryl monostearate (or glyceryl stearate), glyceryl monoisostearate, glyceryl monopalmitate, glyceryl monobehenate; fatty alcohols chosen from non-alkoxylated, saturated or unsaturated, linear or branched fatty alcohols having from 6 to 60 carbon atoms, particularly cetyl alcohol, stearyl alcohol, cetearyl alcohol (mixture of cetyl alcohol and stearyl alcohol), octyldodecanol, 2-butyloctanol, 2-hexyldecanol, 2-undecylpentadecanol, oleic alcohol, linoleic alcohol, behenyl alcohol, 2-dodecylhexadecanol, 2-tetradecyl-1-octadecanol, 2-tetradecyl-1-eicosanol, 2-hexadecyl-1-octadecanol and 2-hexadecyl-1-eicosanol, octyldodecanol, 2-butyloctanol, 2-hexyldecanol, 2-undecylpentadecanol, oleyl alcohol or linoleyl alcohol, isostearyl alcohol, alkoxylated alcohols and lanolin chosen from PEG-40 hydrogenated castor oil, PEG-75 lanolin, laureth-7, laureth-12, trideceth-10, trideceth-12; alkylpolyglucosides chosen from cetearyl glucoside, decyl glucoside, lauryl glucoside, stearyl glucoside, coco-glucoside; or mixtures thereof.

8. The cosmetic composition according to any one of the preceding claims, further comprising at least one non-latex nonionic film forming polymer, wherein the at least one non-latex nonionic film forming polymer is present in an amount ranging from 0.05% to 15% by weight, preferably from 0.5% to 5% by weight, relative to the total weight of the composition.

9. The cosmetic composition according to any one of the preceding claims, further comprising an additional thickening agent other than the thickening agent in claim 1 present in a total amount ranging from 0.05% to 10% by weight, relative to the total weight of the composition; at least one cosmetically acceptable organic solvent; or both,

10. The cosmetic composition according to claim 1 comprising:
at least one acrylate latex polymer;
at least one polyurethane latex polymer;
at least one aminofunctional silicone chosen from amodimethicone/morpholinomethyl silsesquioxane copolymer, PEG-401PPG-8 methylaminopropyl/hydroxypropyl dimethicone copolymer, trideceth-9 PG-amodimethicone, or mixtures thereof, and present in an amount ranging from 0.25% to 3% by weight; , preferably wherein the at least one aminofunctional silicone comprises PEG-40/PPG-8 methylaminopropyl/ hydroxypropyl dimethicone copolymer and trideceth-9 PG-amodimethicone, and most preferably comprises trideceth-9 PG-amodimethicone;
at least one thickening agent chosen from agent chosen from optionally crosslinked and/or neutralized 2-acrylamido-2-methylpropanesulfonic acid polymers (AMPS) and copolymers, crosslinked anionic copolymers of acrylamide, crosslinked anionic copolymers of AMPS, emulsions of crosslinked anionic copolymers of acrylamide/nonionic surfactants, emulsions of AMPS/nonionic surfactants, or mixtures thereof, and present in an amount ranging from 0.1% to 8% by weight;
at least one nonionic surfactant chosen from glyceryl esters, fatty alcohols, alkoxylated alcohols and lanolin, alkylpolyglucosides, or mixtures thereof and present in an amount ranging from 0.075% to 7% by weight; and
water;
wherein all weights are relative to the total weight of the composition; and
wherein the weight ratio of the at least one acrylate latex polymer to the at least one polyurethane latex polymer is equal to or less than 1.

11. A method of styling or shaping hair, the method comprising applying to the hair a composition according to any one of the preceding claims.

12. The method of claim 11, including a step of air drying the hair, wherein the method does not include treating the hair with heat during or after applying the composition onto the hair.

13. The method of claim 11, further comprising a step of treating the hair with heat at a temperature ranging from 25°C to 250°C before, during, or after the application of said composition.

## Patentansprüche

1. Kosmetische Zusammensetzung, umfassend:
wenigstens zwei Latexpolymere, wobei wenigstens ein Latexpolymer ein filmbildendes Polymer ist;
wenigstens ein aminofunktionelles Silicon, ausgewählt aus Amodimethicone/Morpholinomethyl Silsesquioxane Copolymer, PEG-40/PPG-8 Methylaminopropyl/Hydroxypropyl Dimethicone Copolymer, Trideceth-9 PG-Amodimethicone oder Gemischen daraus;
wenigstens ein Verdickungsmittel, ausgewählt aus optional vernetzten und/oder neutralisierten 2-Acrylamido-2-methylpropansulfonsäure-Polymeren (AMPS) und -Copolymeren, vernetzten anionischen Copolymeren von Acrylamid, vernetzten anionischen Copolymeren von AMPS, Emulsionen aus vernetzten anionischen Copolymeren von Acrylamid/nichtionischen Tensiden, Emulsionen aus AMPS/nichtionischen Tensiden oder Gemischen daraus;
wenigstens ein nichtionisches Tensid, ausgewählt aus Glycerinestern, Fettalkoholen, alkoxylierten Alkoholen und Lanolin, Alkylpolyglucosiden oder Gemischen daraus; und
Wasser;
wobei die wenigstens zwei Latexpolymere in einer kombinierten Menge im Bereich von 0,1 Gew.-% bis 30 Gew.-%, insbesondere von 0,25 Gew.-% bis 20 Gew.-%, bezogen auf das Gewicht der Zusammensetzung, vorliegen und
wobei das wenigstens eine aminofunktionelle Silicon in einer Menge im Bereich von 0,25 Gew.-% bis 3 Gew.-%, bezogen auf das Gesamtgewicht der Zusammensetzung, vorliegt.

2. Kosmetische Zusammensetzung nach Anspruch 1, wobei die wenigstens zwei Latexpolymere wenigstens ein Acrylat-Latexpolymer und wenigstens ein Polyurethan-Latexpolymer umfassen, wobei die wenigstens zwei Latexpolymere vorzugsweise Acrylates Copolymer und Polyurethane-34 umfassen.

3. Kosmetische Zusammensetzung nach Anspruch 2, wobei das Gewichtsverhältnis des wenigstens einen Acrylat-Latexpolymers zu dem wenigstens einen Polyurethan-Latexpolymer im Bereich von 10:1 bis 1:10, insbesondere von 5:1 bis 1:5, liegt, ganz besonders 1 beträgt.

4. Kosmetische Zusammensetzung nach einem der vorhergehenden Ansprüche, wobei das wenigstens eine Verdickungsmittel in einer Menge im Bereich von 0,05 Gew.-% bis 10 Gew.-%, bezogen auf das Gesamtgewicht der Zusammensetzung, insbesondere von 0,3 Gew.-% bis 5 Gew.-%, bezogen auf das Gesamtgewicht der Zusammensetzung, vorliegt.

5. Kosmetische Zusammensetzung nach einem der vorhergehenden Ansprüche, wobei das wenigstens eine Verdickungsmittel ausgewählt ist aus einer Wasser-in-Öl-Emulsion aus Polyacrylamide/C13-14 Isoparaffin/Laureth-7, einer Wasser-in-Öl-Emulsion aus Acrylamide/Sodium Acryloyldimethyltaurate Copolymer/Isohexadecane/Polysorbate 80 oder Gemischen daraus.

6. Kosmetische Zusammensetzung nach einem der vorhergehenden Ansprüche, wobei das wenigstens eine nichtionische Tensid in einer Menge im Bereich von 0,05 Gew.-% bis 8 Gew.-%, bezogen auf das Gesamtgewicht der Zusammensetzung, insbesondere von 0,3 Gew.-% bis 5 Gew.-%, bezogen auf das Gesamtgewicht der Zusammensetzung, vorliegt.

7. Kosmetische Zusammensetzung nach einem der vorhergehenden Ansprüche, wobei das wenigstens eine nichtionische Tensid umfasst: Glycerinester, ausgewählt aus Glycerinoleat, Glycerinmonostearat (oder Glycerinstearat), Glycerinmonoisostearat, Glycerinmonopalmitat, Glycerinmonobehenat; Fettalkohole, ausgewählt aus nichtalkoxylierten, gesättigten oder ungesättigten, linearen oder verzweigten Fettalkoholen mit 6 bis 60 Kohlenstoffatomen, insbesondere Cetylalkohol, Stearylalkohol, Cetearylalkohol (Gemisch aus Cetylalkohol und Stearylalkohol), Octyldodecanol, 2-Butyloctanol, 2-Hexyldecanol, 2-Undecylpentadecanol, Oleylalkohol, Linoleylalkohol, Behenylalkohol, 2-Dodecylhexadecanol, 2-Tetradecyl-1-octadecanol, 2-Tetradecyl-1-eicosanol, 2-Hexadecyl-1-octadecanol und 2-Hexadecyl-1-eicosanol, Octyldodecanol, 2-Butyloctanol, 2-Hexyldecanol, 2-Undecylpentadecanol, Oleylalkohol oder Linoleylalkohol, Isostearylalkohol; alkoxylierte Alkohole und Lanolin, ausgewählt aus PEG-40 Hydrogenated Castor Oil, PEG-75 Lanolin, Laureth-7, Laureth-12, Trideceth-10, Trideceth-12; Alkylpolyglucoside, ausgewählt aus Cetearylglucosid, Decylglucosid, Laurylglucosid, Stearylglucosid, Cocoglucosid; oder Gemische daraus.

8. Kosmetische Zusammensetzung nach einem der vorhergehenden Ansprüche, ferner umfassend wenigstens ein nichtionisches filmbildendes Nichtlatex-Polymer, wobei das wenigstens eine nichtionische filmbildende Nichtlatex-Polymer in einer Menge im Bereich von 0,05 Gew.-% bis 15 Gew.-%, vorzugsweise von 0,5 Gew.-% bis 5 Gew.-%, bezogen auf das Gesamtgewicht der Zusammensetzung, vorliegt.

9. Kosmetische Zusammensetzung nach einem der vorhergehenden Ansprüche, ferner umfassend: ein zusätzliches Verdickungsmittel, bei dem es sich nicht um das Verdickungsmittel in Anspruch 1 handelt und das in einer Gesamtmenge im Bereich von 0,05 Gew.-% bis 10 Gew.-%, bezogen auf das Gesamtgewicht der Zusammensetzung, vorliegt; wenigstens ein kosmetisch akzeptables organisches Lösemittel; oder beide.

10. Kosmetische Zusammensetzung nach Anspruch 1, umfassend:
wenigstens ein Acrylat-Latexpolymer;
wenigstens ein Polyurethan-Latexpolymer;
wenigstens ein aminofunktionelles Silicon, ausgewählt aus Amodimethicone/Morpholinomethyl Silsesquioxane Copolymer, PEG-40/PPG-8 Methylaminopropyl/Hydroxypropyl Dimethicone Copolymer, Trideceth-9 PG-Amodimethicone oder Gemischen daraus und in einer Menge im Bereich von 0,25 Gew.-% bis 3 Gew.-% vorliegend, wobei das wenigstens eine aminofunktionelle Silicon vorzugsweise PEG-40/PPG-8 Methylaminopropyl/Hydroxypropyl Dimethicone Copolymer und Trideceth-9 PG-Amodimethicone umfasst und ganz besonders bevorzugt Trideceth-9 PG-Amodimethicone umfasst;
wenigstens ein Verdickungsmittel, ausgewählt aus einem Agens ausgewählt aus optional vernetzten und/oder neutralisierten 2-Acrylamido-2-methylpropansulfonsäure-Polymeren (AMPS) und -Copolymeren, vernetzten anionischen Copolymeren von Acrylamid, vernetzten anionischen Copolymeren von AMPS, Emulsionen aus vernetzten anionischen Copolymeren von Acrylamid/nichtionischen Tensiden, Emulsionen aus AMPS/nichtionischen Tensiden oder Gemischen daraus und in einer Menge im Bereich von 0,1 Gew.-% bis 8 Gew.-% vorliegend;
wenigstens ein nichtionisches Tensid, ausgewählt aus Glycerinestern, Fettalkoholen, alkoxylierten Alkoholen und Lanolin, Alkylpolyglucosiden oder Gemischen daraus und in einer Menge im Bereich von 0,075 Gew.-% bis 7 Gew.-% vorliegend; und
Wasser;
wobei alle Gewichtsangaben sich auf das Gesamtgewicht der Zusammensetzung beziehen; und
wobei das Gewichtsverhältnis des wenigstens einen Acrylat-Latexpolymers zu dem wenigstens einen Polyurethan-Latexpolymer gleich oder kleiner als 1 ist.

11. Verfahren zum Stylen oder Formen von Haar, wobei das Verfahren ein Auftragen einer Zusammensetzung nach einem der vorhergehenden Ansprüche auf das Haar umfasst.

12. Verfahren nach Anspruch 11, umfassend einen Schritt des Lufttrocknens des Haars, wobei das Verfahren kein Behandeln des Haars mit Wärme während oder nach dem Auftragen der Zusammensetzung auf das Haar umfasst.

13. Verfahren nach Anspruch 11, das ferner einen Schritt des Behandelns des Haars mit Wärme bei einer Temperatur im Bereich von 25 °C bis 250 °C vor, während oder nach dem Auftragen der Zusammensetzung umfasst.

## Revendications

1. Composition cosmétique comprenant :
au moins deux polymères de latex, dans laquelle au moins un polymère de latex est un polymère filmogène ;
au moins une silicone aminofonctionnelle choisie parmi un copolymère amodiméthicone/morpholinométhyl silsesquioxane, un copolymère PEG-40/PPG-8 méthylaminopropyl/hydroxypropyl diméthicone, un tridéceth-9 PG-amodiméthicone, ou des mélanges de ceux-ci ;
au moins un agent épaississant choisi parmi des polymères et copolymères d'acide 2-acrylamido-2-méthylpropanesulfonique (AMPS) éventuellement réticulés et/ou neutralisés, des copolymères anioniques réticulés d'acrylamide, des copolymères anioniques réticulés d'AMPS, des émulsions de copolymères anioniques réticulés d'acrylamide/tensioactifs non ioniques, des émulsions d'AMPS/tensioactifs non ioniques, ou des mélanges de ceux-ci ;
au moins un tensioactif non ionique choisi parmi les esters de glycéryle, les alcools gras, les alcools alcoxylés et la lanoline, les alkylpolyglucosides, ou les mélanges de ceux-ci ; et
de l'eau ;
dans laquelle les au moins deux polymères de latex sont présents en une quantité combinée dans la plage de 0,1 % à 30 % en poids, notamment de 0,25 % à 20 % en poids, par rapport au poids de la composition, et
dans laquelle l'au moins une silicone aminofonctionnelle est présente en une quantité dans la plage de 0,25 % à 3 % en poids, par rapport au poids total de la composition.

2. Composition cosmétique selon la revendication 1, dans laquelle les au moins deux polymères de latex comprennent au moins un polymère de latex d'acrylate et au moins un polymère de latex de polyuréthane, de préférence dans laquelle les au moins deux polymères de latex comprennent un copolymère d'acrylates et du polyuréthane-34.

3. Composition cosmétique selon la revendication 2, dans laquelle le rapport en poids de l'au moins un polymère de latex d'acrylate sur l'au moins un polymère de latex de polyuréthane se trouve dans la plage de de 10/1 à 1/10, particulièrement de 5/1 à 1/5, plus particulièrement de 1.

4. Composition cosmétique selon l'une quelconque des revendications précédentes, dans laquelle l'au moins un agent épaississant est présent en une quantité dans la plage de 0,05 % à 10 % en poids, par rapport au poids total de la composition, notamment de 0,3 % à 5 % en poids, par rapport au poids total de la composition.

5. Composition cosmétique selon l'une quelconque des revendications précédentes, dans laquelle l'au moins un agent émulsifiant est sélectionné parmi une émulsion eaudans-1'huile de polyacrylamide/isoparaffine en C13-14/laureth-7, une émulsion eau-dans-l'huile d'acrylamide/copolymère d'acryloyldiméthyltaurate de sodium/isohexadécane/polysorbate 80 ou des mélanges de celles-ci.

6. Composition cosmétique selon l'une quelconque des revendications précédentes, dans laquelle l'au moins un tensioactif non ionique est présent en une quantité dans la plage de 0,05 % à 8 % en poids, par rapport au poids total de la composition, particulièrement de 0,3 % à 5 % en poids, par rapport au poids total de la composition.

7. Composition cosmétique selon l'une quelconque des revendications précédentes, dans laquelle l'au moins un tensioactif non ionique inclut des esters de glycéryle choisis parmi l'oléate de glycéryle, le monostéarate de glycéryle (ou le stéarate de glycéryle), le monoisostéarate de glycéryle, le monopalmitate de glycéryle, le monobéhénate de glycéryle ; des alcools gras choisis parmi des alcools gras linéaires ou ramifiés, saturés ou insaturés, non alcoxylés ayant de 6 à 60 atomes de carbone notamment l'alcool cétylique, l'alcool stéarylique, l'alcool cétéarylique (un mélange d'alcool cétylique et d'alcool stéarylique), l'octyldodécanol, le 2-butyloctanol, le 2-hexyldécanol, le 2-undécylpentadécanol, l'alcool oléique, l'alcool linoléique, l'alcool béhénylique, le 2-dodécylhexadécanol, le 2-tétradécyl-1-octadécanol, le 2-tétradécyl-1-éicosanol, le 2-hexadécyl-1-octadécanol et le 2-hexadécyl-1-éicosanol, l'octyldodécanol, le 2-butyloctanol, le 2-hexyldécanol, le 2-undécylpentadécanol, l'alcool oléylique ou l'alcool linoléylique, l'alcool isostéarylique ; des alcools alcoxylés et de la lanoline choisis parmi l'huile de ricin hydrogénée PEG-40, la lanoline PEG-75, le laureth-7, le laureth-12, le tridéceth-10, le tridéceth-12 ; des alkylpolyglucosides choisis parmi le cétéaryl glucoside, le décyl glucoside, le lauryl glucoside, le stéaryl glucoside, le coco-glucoside ; ou des mélanges de ceux-ci.

8. Composition cosmétique selon l'une quelconque des revendications précédentes, comprenant en outre au moins un polymère filmogène non ionique non latex, dans laquelle l'au moins un polymère filmogène non ionique non latex est présent en une quantité dans la plage de 0,05 % à 15 % en poids, de préférence de 0,5 % à 5 % en poids, par rapport au poids total de la composition.

9. Composition cosmétique selon l'une quelconque des revendications précédentes, comprenant en outre un agent épaississant supplémentaire autre que l'agent épaississant de la revendication 1 présent en une quantité totale dans la plage de 0,05 % à 10 % en poids, par rapport au poids total de la composition ; au moins un solvant organique acceptable sur le plan cosmétique ; ou les deux.

10. Composition cosmétique selon la revendication 1 comprenant :
au moins un polymère de latex d'acrylate ;
au moins un polymère de latex de polyuréthane ;
au moins une silicone aminofonctionnelle choisie parmi un copolymère d'amodiméthicone/morpholinométhyl silsesquioxane, un copolymère PEG-40/PPG-8 méthylaminopropyl/hydroxypropyl diméthicone, un tridéceth-9 PG-amodiméthicone, ou des mélanges de ceux-ci, et présent en une quantité dans la plage de 0,25 % à 3 % en poids ; de préférence dans laquelle l'au moins une silicone aminofonctionnelle comprend un copolymère PEG-40/PPG-8 méthylaminopropyl/hydroxypropyl diméthicone et un tridéceth-9 PG-amodiméthicone, et de manière préférée entre toute comprend un tridéceth-9 PG-amodiméthicone ;
au moins un agent épaississant choisi parmi un agent choisi parmi des polymères et copolymères d'acide 2-acrylamido-2-méthylpropanesulfonique (AMPS) éventuellement réticulés et/ou neutralisés, des copolymères anioniques réticulés d'acrylamide, des copolymères anioniques réticulés d'AMPS, des émulsions de copolymères anioniques réticulés d'acrylamide/tensioactifs non ioniques, des émulsions d'AMPS/tensioactifs non ioniques, ou des mélanges de ceux-ci ; et présent en une quantité dans la plage de 0,1 % à 8 % en poids ;
au moins un tensioactif non ionique choisi parmi les esters de glycéryle, les alcools gras, les alcools alcoxylés et la lanoline, les alkylpolyglucosides ou des mélanges de ceux-ci présents en une quantité dans la plage de 0,075 % à 7 % en poids ; et
de l'eau ;
dans laquelle tous les poids sont par rapport au poids total de la composition ; et
dans laquelle le rapport en poids de l'au moins un polymère de latex d'acrylate sur l'au moins un polymère de latex de polyuréthane est inférieur ou égal à 1.

11. Procédé de mise en forme ou de coiffage de cheveux, le procédé comprenant l'application aux cheveux d'une composition selon l'une quelconque des revendications précédentes.

12. Procédé selon la revendication 11, incluant une étape de séchage à l'air des cheveux dans lequel le procédé n'inclut pas le traitement des cheveux avec de la chaleur pendant ou après l'application de la composition sur les cheveux.

13. Procédé selon la revendication 11, comprenant en outre une étape de traitement des cheveux avec de la chaleur à une température dans une plage de 25 °C à 250 °C avant, pendant ou après l'application de ladite composition.
